(19) Europäisches Patentamt
European Patent Office
Office européen des brevets

(11) EP 4 285 936 A1

(12) EUROPEAN PATENT APPLICATION
published in accordance with Art. 153(4) EPC

(43) Date of publication:
**06.12.2023 Bulletin 2023/49**

(21) Application number: **22745259.6**

(22) Date of filing: **26.01.2022**

(51) International Patent Classification (IPC):
***A61K 47/68*** *(2017.01)* ***C07K 16/18*** *(2006.01)*
***A61K 39/00*** *(2006.01)* ***A61P 35/00*** *(2006.01)*
***A61P 37/02*** *(2006.01)*

(52) Cooperative Patent Classification (CPC):
**A61K 47/68037; A61K 47/6849; A61K 47/6889; A61P 35/00; C07K 16/2878;** C07K 2317/24; C07K 2317/73; C07K 2317/77

(86) International application number:
**PCT/CN2022/073943**

(87) International publication number:
**WO 2022/161385 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.01.2021 CN 202110123809**

(71) Applicants:
- **Shanghai Hansoh Biomedical Co., Ltd.**
  **Shanghai 201203 (CN)**
- **Jiangsu Hansoh Pharmaceutical Group Co., Ltd.**
  **Lianyungang, Jiangsu 222047 (CN)**

(72) Inventors:
- **HUA, Haiqing**
  **Shanghai 201203 (CN)**
- **MAO, Dongjie**
  **Shanghai 201203 (CN)**
- **LUO, Yan**
  **Shanghai 201203 (CN)**
- **XIE, Yuejun**
  **Shanghai 201203 (CN)**

(74) Representative: **EP&C**
**P.O. Box 3241**
**2280 GE Rijswijk (NL)**

Remarks:
The complete document including Reference Table(s) and the Sequence Listing(s) can be downloaded from the EPO website

# (54) ANTIBODY-DRUG CONJUGATE AND MEDICAL USE THEREOF

(57) An anti-BCMA antibody-drug conjugate, or a pharmaceutically acceptable salt or solvent compound thereof, and the medical use thereof. Specifically, the antibody-drug conjugate is formed by connecting an anti-BCMA antibody or an antigen-binding fragment thereof and an exatecan derivative by using a linker, and the antibody-drug conjugate or the pharmaceutically acceptable salt or solvent compound thereof has a significant anti-tumor effect and good safety.

EP 4 285 936 A1

## Description

The present application claims the priority of the patent application No. 202110123809.3 filed on January 29, 2021.

## FIELD OF THE INVENTION

The present disclosure relates to the field of biomedicine and specifically the present disclosure relates to an anti-BCMA antibody drug conjugate and medical use thereof.

## BACKGROUND OF THE INVENTION

B cells are lymphocytes, which play an important role in adaptive humoral immunity and the production of antibodies that specifically recognize antigens. The three subtypes of B cells are naive B cells, memory B cells and plasma cells. In the process of VDJ recombination, two or three fragments of DNA selected from a genomic library are recombined to produce a combinatorial array of antibody variable domains, thereby at most $10^9$ unique B cell lineages are generated by further altering the variable domains encoded by B cells of different lineages, resulting in antibodies specific for unique targets. B cells are involved in many diseases. Malignant transformation of B cells lead to cancer, including some lymphomas, such as multiple myeloma and Hodgkin's lymphoma. B cells are also involved in autoimmune diseases, including systemic lupus erythematosus (SLE) and IgA nephropathy. B cell-related cancers and autoimmune diseases can be considered as abnormality of B cell function, thus a possible strategy to control such diseases is to use antibodies that target pathological B cells.

BCMA is a member of the TNF receptor superfamily, which is a non-glycosylated intrinsic membrane receptor for the ligands BAFF (a B-cell activator) and APRIL (a proliferation-inducing ligand). BCMA and its corresponding ligands can regulate different functions of humoral immunity, B cell development and homeostasis. BCMA is expressed by tonsillar memory B cells and germinal center B cells, it is detected in the spleen, lymph node, thymus, adrenal gland and liver, and a variety of analysis of B cell lineages show that the expression level of BCMA increases after maturation.

## SUMMARY OF THE INVENTION

The objective of the present disclosure is to provide an antibody-drug conjugate of general formula (I) or a pharmaceutically acceptable salt or solvate thereof,

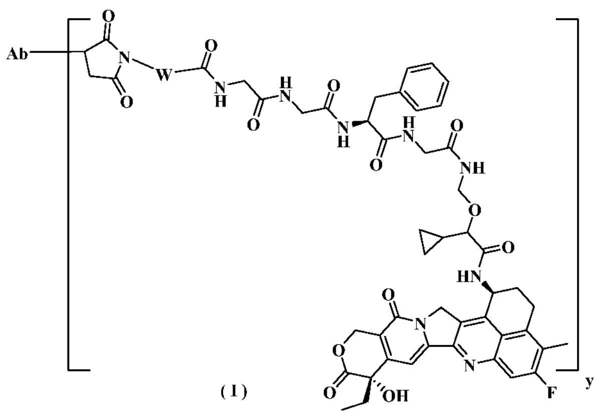

(I)

wherein:

W is $-(CR^eR^f)_g-X_1-(CR^eR^f)_u-X_2-(CR^eR^f)_h-$;

$R^e$ or $R^f$ is independently selected from the group consisting of hydrogen, deuterium, hydroxyl, amino, alkyl, halogen, haloalkyl, deuterated alkyl, hydroxyalkyl; preferably, $R^e$ or $R^f$ is independently selected from the group consisting of hydrogen, deuterium; more preferably, $R^e$ or $R^f$ is hydrogen;

$X_1$ or $X_2$ is independently selected from the group consisting of N, H, O and S; preferably, $X_1$ or $X_2$ is independently selected from S; more preferably, $X_1$ or $X_2$ is independently selected from O;

g, u or h is independently selected from the group consisting of 1, 2, 3 and 4; preferably, g, u or h is independently selected from 1, 2, 3; more preferably, g, u or h is 2;

y is 1-20; preferably, y is 4-10; more preferably, y is 4, 6, 8 or 10;
Ab is an anti-BCMA antibody or an antigen-binding fragment thereof.

In a preferred embodiment of the present disclosure, the anti-BCMA antibody or the antigen-binding fragment thereof comprises a heavy chain variable region and a light chain variable region, wherein the heavy chain variable region comprises an HCDR1, an HCDR2 and an HCDR3 set forth in SEQ ID NO: 3, SEQ ID NO: 4 and SEQ ID NO: 5, respectively, and the light chain variable region comprises an LCDR1, an LCDR2 and an LCDR3 set forth in SEQ ID NO: 6, SEQ ID NO: 7 and SEQ ID NO: 8, respectively.
In a preferred embodiment of the present disclosure, the anti-BCMA antibody or the antigen-binding fragment thereof is a murine antibody, a chimeric antibody, a human antibody, or a humanized antibody.
In a preferred embodiment of the present disclosure, the anti-BCMA antibody or the antigen-binding fragment thereof further comprises a heavy chain constant region derived from human IgG1, IgG2, IgG3 or IgG4 or variant thereof;
Preferably, the anti-BCMA antibody or the antigen-binding fragment thereof further comprises a heavy chain constant region derived from human IgG1, IgG2, or IgG4;
More preferably, the anti-BCMA antibody or the antigen-binding fragment thereof further comprises IgG1 heavy chain constant region with enhanced ADCC toxicity after amino acid mutation; alternatively, the anti-BCMA antibody or the antigen-binding fragment thereof further comprises heavy chain constant region as shown in SEQ ID NO: 22.
In a preferred embodiment of the present disclosure, the anti-BCMA antibody or the antigen-binding fragment thereof further comprises a light chain constant region derived from human κ chain, λ chain or variant thereof; preferably, the anti-BCMA antibody or the antigen-binding fragment thereof further comprises a light chain constant region derived from human κ chain; more preferably, the anti-BCMA antibody or the antigen-binding fragment thereof further comprises a light chain constant region as shown in SEQ ID NO: 23.
In a preferred embodiment of the present disclosure, the anti-BCMA antibody or the antigen-binding fragment thereof comprises a heavy chain variable region selected from the group consisting of SEQ ID NO: 9, SEQ ID NO: 10 and SEQ ID NO: 11, or a heavy chain variable region having at least 70%, 75%, 80%, 85%, 90%, 95% or 99% identity with the same;
and/or the anti-BCMA antibody or the antigen-binding fragment thereof comprises a light chain variable region selected from the group consisting of SEQ ID NO: 12, SEQ ID NO: 13 and SEQ ID NO: 14, or a light chain variable region having at least 70%, 75%, 80%, 85%, 90%, 95% or 99% identity with the same.
In a preferred embodiment of the present disclosure, the anti-BCMA antibody or the antigen-binding fragment thereof comprises a heavy chain selected from the group consisting of SEQ ID NO: 15, SEQ ID NO: 16 and SEQ ID NO: 17, or a heavy chain having at least 80%, 85%, 90%, 95% or 99% identity with the same.
and/or the anti-BCMA antibody or the antigen-binding fragment thereof comprises a light chain selected from the group consisting of SEQ ID NO: 18, SEQ ID NO: 19 and SEQ ID NO: 20, or a light chain having at least 80%, 85%, 90%, 95% or 99% identity with the same.
In a preferred embodiment of the present disclosure, the anti-BCMA antibody or the antigen-binding fragment thereof comprises a heavy chain variable region as shown in SEQ ID NO: 9 and a light chain variable region as shown in SEQ ID NO: 12; or

the anti-BCMA antibody or the antigen-binding fragment thereof comprises a heavy chain variable region as shown in SEQ ID NO: 10 and a light chain variable region as shown in SEQ ID NO: 13; or
the anti-BCMA antibody or the antigen-binding fragment thereof comprises a heavy chain variable region as shown in SEQ ID NO: 11 and a light chain variable region as shown in SEQ ID NO: 14.

In a preferred embodiment of the present disclosure, the anti-BCMA antibody comprises a heavy chain as shown in SEQ ID NO: 15 and a light chain as shown in SEQ ID NO: 18, or

the anti-BCMA antibody comprises a heavy chain as shown in SEQ ID NO: 16 and a light chain as shown in SEQ ID NO: 19, or
the anti-BCMA antibody comprises a heavy chain as shown in SEQ ID NO: 17 and a light chain as shown in SEQ ID NO: 20.

In a preferred embodiment of the present disclosure, the antibody drug conjugate or the pharmaceutically acceptable salt or solvate thereof is as shown in general formula (II), or a tautomer, mesomer, racemate, enantiomer, diastereomer or mixture form thereof;

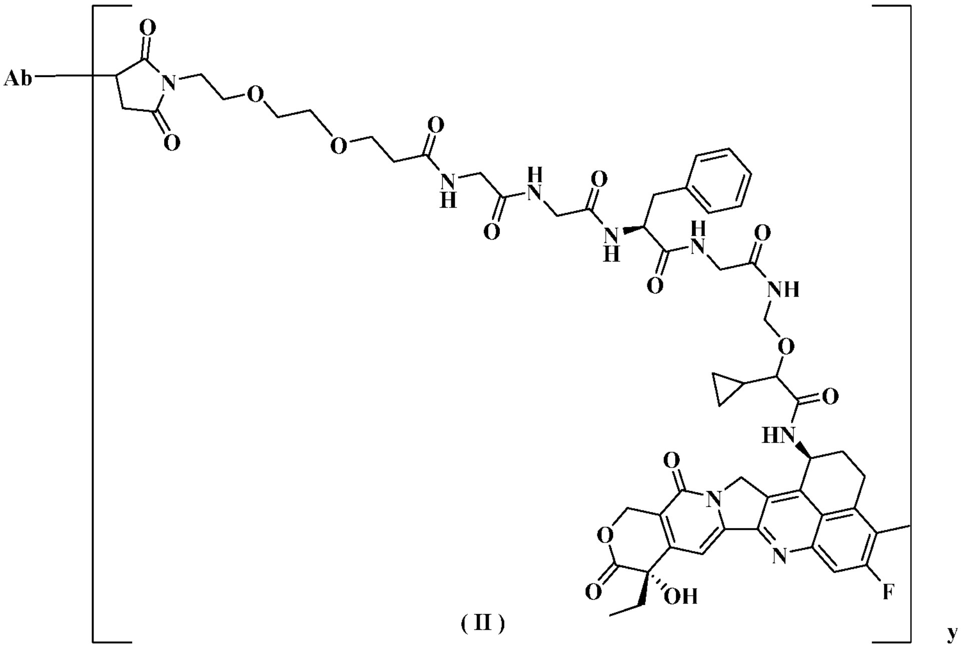

( II )

wherein Ab is the anti-BCMA antibody or the antigen-binding fragment as defined above,
y is a number selected from 2 to 10, preferably a number selected from 4 to 10, more preferably, 4, 6, 8 or 10.

In a preferred embodiment of the present disclosure, the antibody drug conjugate or the pharmaceutically acceptable salt or solvate is as shown in general formula (III) or a pharmaceutically acceptable salt or solvate thereof;

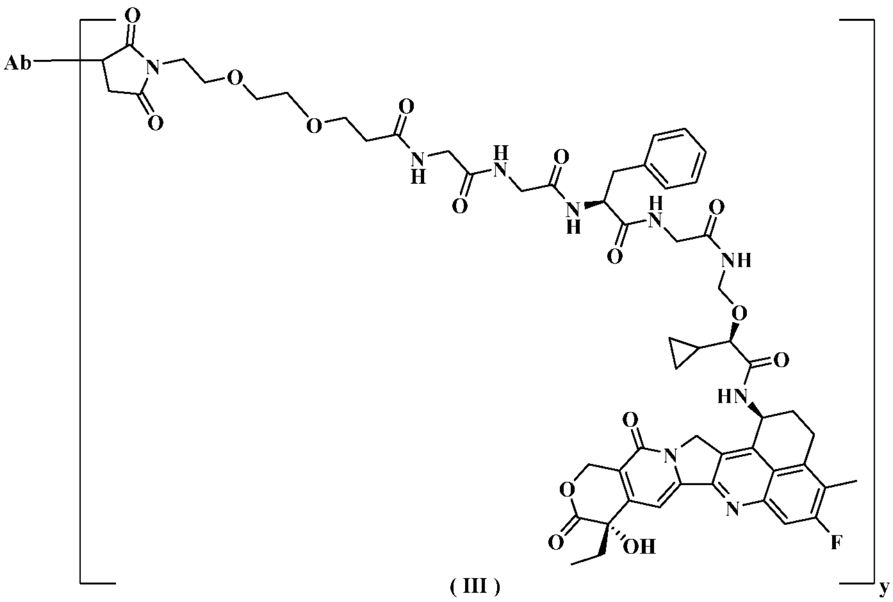

( III )

wherein Ab is the anti-BCMA antibody or the antigen-binding fragment as defined above,
y is a number selected from 2 to 10, preferably a number selected from 4 to 10, more preferably, 4, 6, 8 or 10.

In a preferred embodiment of the present disclosure, the antibody drug conjugate or the pharmaceutically acceptable salt or solvate is selected from the group consisting of the following structures:

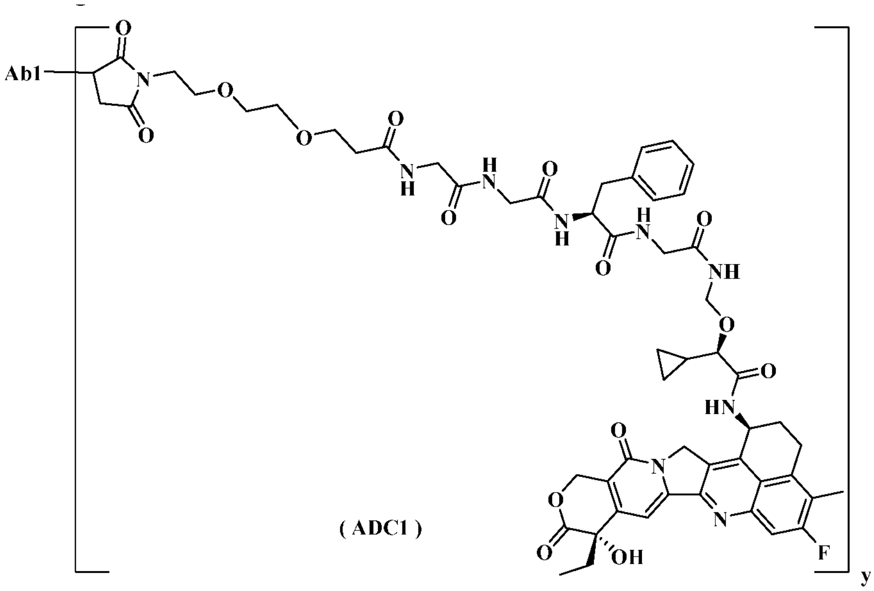

( ADC1 )

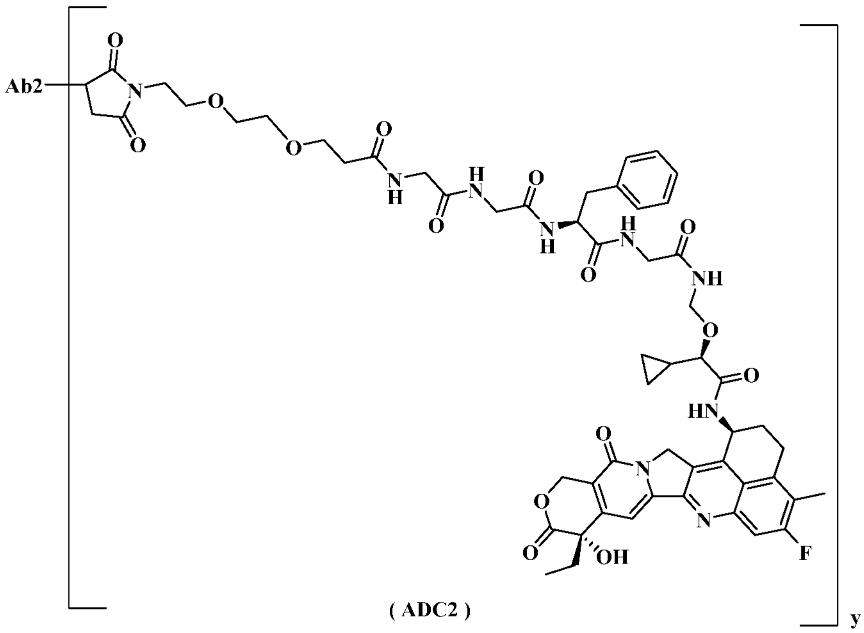

( ADC2 )

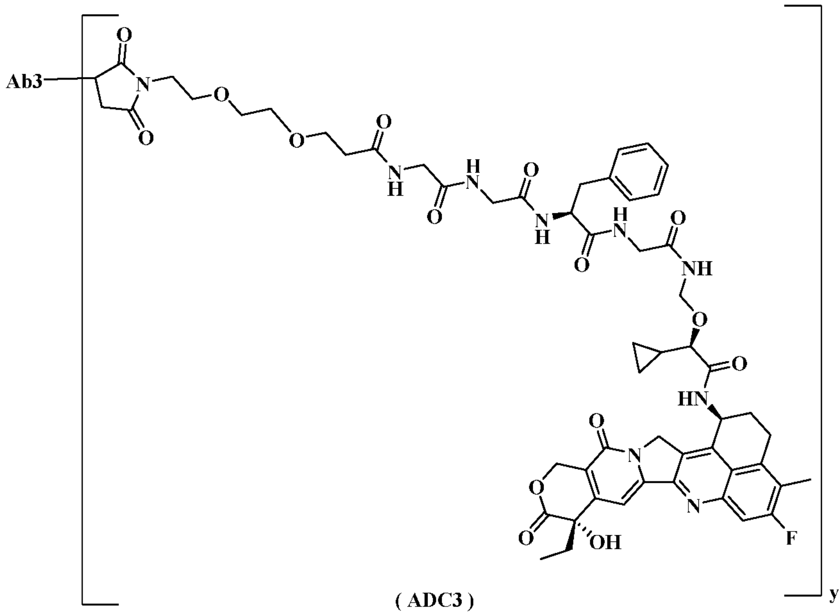

( ADC3 )

wherein, y is a number selected from 2 to 10, preferably a number selected from 4 to 10, more preferably, 4, 6, 8 or 10.

The present disclosure also provides a method for preparing the antibody drug conjugate of general formula (I) or the pharmaceutically acceptable salt or solvate thereof, which comprises the following steps:

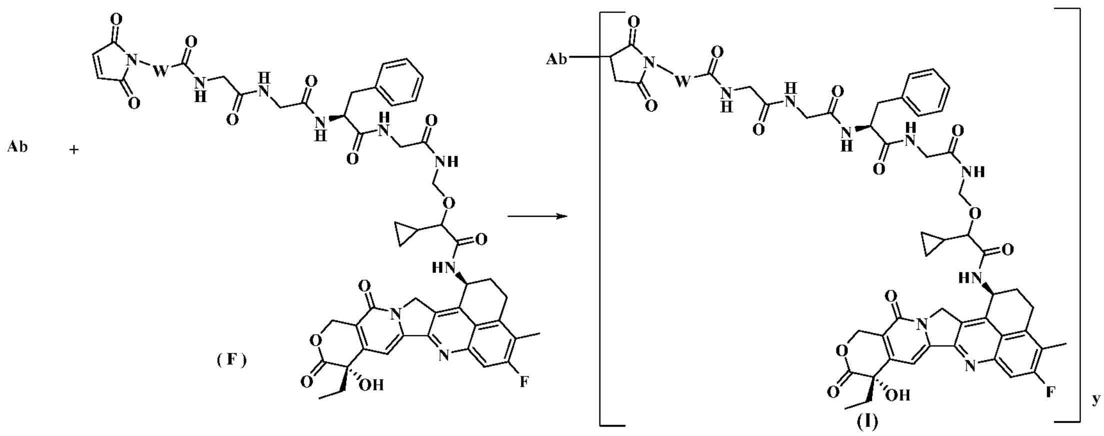

after Ab is reduced, it is subjected to coupling reaction with general formula (F) to obtain the compound of general formula (I);

wherein: W is as defined above.

Ab is the anti-BCMA antibody or the antigen-binding fragment as defined above,

y is a number selected from 1 to 20, preferably a number selected from 4 to 10, more preferably, 4, 6, 8 or 10.

In another aspect, the present disclosure provides a pharmaceutical composition comprising the above mentioned antibody drug conjugate or the pharmaceutically acceptable salt or solvate thereof, and one or more pharmaceutically acceptable excipients, diluents or carriers.

In another aspect, the present disclosure relates to use of the above mentioned antibody drug conjugate or the pharmaceutically acceptable salt or solvate thereof, or the above mentioned pharmaceutical composition in the preparation of a medicament for treating or preventing a BCMA-mediated disease or condition.

In a preferred embodiment, the BCMA-mediated disease or condition is cancer or autoimmune disease; wherein the cancer is preferably a cancer expressing BCMA, more preferably lymphoma, leukemia or myeloma, and the autoimmune disease is preferably selected from the group consisting of lupus erythematosus, IgA nephropathy and rheumatic arthritis.

The antibody drug conjugate or pharmaceutically acceptable salt or solvate thereof of the present disclosure have significant anti-tumor effect and good safety, and have good metabolic activity in vivo, long drug duration *in vivo*,

and broad prospects for clinical application.

## DETAILED DESCRIPTION OF THE INVENTION

### Terms

To make it easier to understand the present disclosure, certain technical and scientific terms are specifically defined below. Unless otherwise clearly defined elsewhere herein, all other technical and scientific terms used herein have the meanings commonly understood by those skilled in the art to which the present disclosure belongs.

The three-letter codes and one-letter codes of amino acids used in the present disclosure are as described in J. Biol. Chem, 243, p3558(1968).

The term "antibody" described in the present disclosure refers to an immunoglobulin, which is a tetrapeptide chain structure consisting of two identical heavy chains and two identical light chains linked by interchain disulfide bonds. The amino acid composition and order of arrangement of the immunoglobulin heavy chain constant regions are different, so their antigenicity is also different. According to this, immunoglobulins can be classified into five types, or known as isotypes of immunoglobulins, namely IgM, IgD, IgG, IgA and IgE, and their corresponding heavy chains are μ chain, δ chain, y chain, α chain and ε chain, respectively. The same type of Ig can be classified into different subclasses according to the difference in the amino acid composition of the hinge region and the number and position of heavy chain disulfide bonds. For example, IgG can be classified into IgG1, IgG2, IgG3 and IgG4. The light chain is classified into κ chain or λ chain according to the difference in the constant region. Each of the five types of Ig can have κ chain or λ chain.

In the present disclosure, the antibody light chain variable region according to the present disclosure can further comprise a light chain constant region, which comprises human or murine κ, λ chain or variant thereof.

In the present disclosure, the antibody heavy chain variable region according to the present disclosure can further comprise heavy chain constant region, which comprises human or murine IgG1, IgG2, IgG3, IgG4 or variant thereof.

The sequence of about 110 amino acids near the N-terminus of the antibody heavy and light chains varies greatly and is the variable region (V region); the rest amino acid sequence near the C-terminus is relatively stable and is the constant region (C region). The variable region comprises 3 hypervariable regions (HVRs) and 4 framework regions (FRs) with relatively conservative sequences. The 3 hypervariable regions determine the specificity of the antibody, and they are also known as complementarity determining regions (CDRs). Each of the light chain variable region (VL) and heavy chain variable region (VH) consists of 3 CDR regions and 4 FR regions. The order of arrangement from the amino terminus to the carboxyl terminus is: FR1, CDR1, FR2, CDR2, FR3, CDR3, FR4. The 3 CDR regions of the light chain refer to LCDR1, LCDR2 and LCDR3; the 3 CDR regions of the heavy chain refer to HCDR1, HCDR2 and HCDR3. The number and position of the CDR amino acid residues in the VL region and VH region of the antibody or antigen-binding fragment according to the present disclosure comply with the known Kabat numbering criteria and Kabat, AbM or IMGT definition criteria (http://bioinf.org.uk/abs/).

The term "antigen presenting cell" or "APC" is a cell that presents foreign antigen complexed with MHC on its surface. T cells utilize T cell receptors (TCRs) to recognize such complexes. Examples of APCs include, but are not limited to, dendritic cells (DCs), peripheral blood mononuclear cells (PBMCs), monocytes, B lymphoblast and monocyte-derived dendritic cells.

The term "antigen presenting" refers to the process of capturing antigens by APCs and enabling them to be recognized by T cells, for example as a component of MHC-I/MHC-II conjugate.

The term "BCMA" includes any variant or isoform of BCMA that is naturally expressed by cells. The antibodies of the present disclosure can cross-react with BCMA derived from non-human species. Alternatively, the antibodies can also be specific for human BCMA and may not show cross-reactivity with other species. BCMA or any variant or isoform thereof can be isolated from cells or tissues naturally expressing the same, or produced by recombinant techniques using techniques commonly used in the art and those described herein. Preferably, the anti-BCMA antibody targets human BCMA that has a normal glycosylation pattern.

The term "recombinant human antibody" includes human antibodies prepared, expressed, created or isolated by recombinant methods, and the techniques and methods involved are well known in the art, such as:

1. antibodies isolated from transgenic and trans-chromosomal animals (for example mice) with human immunoglobulin genes, or from hybridomas prepared therefrom;
2. antibodies isolated from host cells transformed to express the antibodies, such as transfectoma;
3. antibodies isolated from recombinant combinatorial human antibody libraries; and
4. antibodies prepared, expressed, created or isolated by methods such as splicing human immunoglobulin gene sequences with other DNA sequences.

Such recombinant human antibodies comprise variable region and constant region, which utilize specific human germline immunoglobulin sequences encoded by germline genes, but also comprise subsequent rearrangements and mutations such as those occur during antibody maturation.

The term "murine antibody" in the present disclosure is a monoclonal antibody against human BCMA prepared according to the knowledge and skills in the art. During preparation, the test subject is injected with BCMA antigen, and then hybridomas expressing antibody having the desired sequence or functional properties are isolated. In a preferred embodiment of the present disclosure, the murine BCMA antibody or the antigen-binding fragment thereof may further comprise the light chain constant region of murine κ , λ chain or variant thereof, or further comprise the heavy chain constant region of murine IgG1, IgG2, IgG3 or IgG4 or variant thereof.

The term "human antibody" includes antibodies having variable region and constant region of human germline immunoglobulin sequences. The human antibodies of the present disclosure may include amino acid residues that are not encoded by human germline immunoglobulin sequences (such as mutations introduced by random or site-specific mutagenesis in vitro or by somatic mutations in vivo). However, the term "human antibody" does not include antibodies in which CDR sequences derived from the germline of another mammalian species (such as mice) have been grafted onto human framework sequences (namely "humanized antibodies").

The term "humanized antibody", also known as CDR-grafted antibody, refers to the antibody produced by grafting murine CDR sequences into the frameworks of human antibody variable region. Humanized antibody can overcome the shortcomings of strong immune responses induced by chimeric antibodies that carry a large amount of murine protein components. In order to avoid the decrease in activity caused by the decrease in immunogenicity, the human antibody variable regions can be subjected to minimal reverse mutation to maintain the activity.

The term "chimeric antibody" is an antibody formed by fusing the variable region of a murine antibody with the constant region of a human antibody, which can alleviate the immune response induced by murine antibody. Establishing a chimeric antibody requires first establishing a hybridoma that secretes murine specific monoclonal antibody, then cloning the variable region gene from the murine hybridoma cells, and then cloning the constant region gene of the human antibody as necessary, linking the murine variable region gene with the human constant region gene to form a chimeric gene, which is inserted into a human expression vector, and finally expressing the chimeric antibody molecule in a eukaryotic industrial system or a prokaryotic industrial system. The human antibody constant region can be selected from the heavy chain constant region of human IgG1, IgG2, IgG3 or IgG4 or variant thereof, preferably human IgG1, IgG2 or IgG4 heavy chain constant region, or IgG1 heavy chain constant region with enhanced ADCC (antibody-dependent cell-mediated cytotoxicity) after amino acid mutation.

The term "antigen-binding fragment" refers to antigen-binding fragment of an antibody and antibody analog, which usually comprises at least part of the antigen-binding region or variable region (for example one or more CDRs) of the parental antibody. The antibody fragment retains at least some of the binding specificity of the parental antibody. Generally, when the activity is represented on a mole basis, the antibody fragment retains at least 10% of the parental binding activity. Preferably, the antibody fragment retains at least 20%, 50%, 70%, 80%, 90%, 95% or 100% or more of the binding affinity of the parental antibody to the target. Examples of antigen-binding fragments include, but are not limited to: Fab, Fab', F(ab')2, Fv fragment, linear antibody, single-chain antibody, nanobody, domain antibody and multispecific antibody. Engineered antibody variants are reviewed in Holliger and Hudson, 2005, Nat. Biotechnol. 23: 1126-1136.

The "Fab fragment" consists of one light chain and the CH1 and variable regions of one heavy chain. The heavy chain of a Fab molecule cannot form disulfide bonds with another heavy chain molecule.

The "Fc" region comprises two antibody heavy chain fragments comprising the CH2 and CH3 domains. The two heavy chain fragments are held together by two or more disulfide bonds and through the hydrophobic interaction of the CH3 domain.

The "Fab' fragment" comprises a light chain and a part of a heavy chain comprising the VH domain, the CH1 domain and the region between the CH1 and CH2 domains, so that interchain disulfide bonds can be formed between the two heavy chains of two Fab' fragments, thereby forming a F(ab')2 molecule.

The "F(ab')2 fragment" comprises two light chains and two heavy chains comprising a part of the constant region between the CH1 and CH2 domains, thereby forming interchain disulfide bonds between the two heavy chains. Therefore, the F(ab')2 fragment consists of two Fab' fragments held together by disulfide bonds between the two heavy chains.

The "Fv region" comprises variable regions from both the heavy chain and the light chain, but lacks constant regions.

The term "multispecific antibody" is used in its broadest sense, encompassing antibodies with specificity for multiple epitopes. These multispecific antibodies include, but are not limited to: antibodies comprising heavy chain variable region VH and light chain variable region VL, wherein the VH-VL unit has specificity for multiple epitopes; antibodies with two or more VL and VH regions, each VH-VL unit binds to different targets or different epitopes of the same target; antibodies with two or more single variable regions, each single variable region binds to different targets

or different epitopes of the same target; full-length antibodies, antibody fragments, diabodies, bispecific diabodies and triabodies, covalently or non-covalently linked antibody fragments, and the like.

The term "single-chain antibody" is a single-chain recombinant protein formed by linking the heavy chain variable region VH and light chain variable region VL of an antibody through a linker peptide. It is the minimum antibody fragment with complete antigen binding site.

The term "domain antibody fragment" is an immunoglobulin fragment having immunological functions, which comprises either the heavy chain variable region or the light chain variable region. In some cases, two or more VH regions are covalently linked by a peptide linker to form a bivalent domain antibody fragment. The two VH regions of the bivalent domain antibody fragment can target the same antigen or different antigens.

The term "binding to BCMA" in the present disclosure refers to being able to interact with human BCMA.

The term "antigen-binding site" in the present disclosure refers to a three-dimensional site recognized by the antibody or antigen-binding fragment of the present disclosure.

The term "epitope" refers to a site on an antigen that specifically binds to an immunoglobulin or antibody. Epitopes can be formed by adjacent amino acids or non-adjacent amino acids that are juxtaposed due to tertiary folding of the protein. Epitopes formed by adjacent amino acids are usually maintained after exposure to a denaturing solvent, while epitopes formed by tertiary folding are usually lost after treatment with a denaturing solvent. Epitopes usually comprise at least 3-15 amino acids in a unique spatial conformation. Methods for determining what epitope is bound to a given antibody are well known in the art, including immunoblotting, immunoprecipitation detection analysis and the like. Methods for determining the spatial conformation of an epitope include the techniques in the art and the techniques described herein, for example X-ray crystal analysis, two-dimensional nuclear magnetic resonance and the like.

The terms "specifically binds" and "selectively binds" used in the present disclosure refer to the binding of an antibody to an epitope on a predetermined antigen. Generally, when recombinant human BCMA is used as the analyte and an antibody is used as the ligand, when measured by surface plasmon resonance (SPR) technology in an instrument, the antibody binds to the predetermined antigen at an equilibrium dissociation constant ($K_D$) of about less than $10^{-7}$ M or even less, and its binding affinity to the predetermined antigen is at least twice as much as its binding affinity to nonspecific antigens (such as BSA and the like) other than the predetermined antigen or closely related antigens. The term "antibody that recognizes antigen" can be used interchangeably with the term "antibody that specifically binds to" herein.

The term "cross-reaction" refers to the ability of the antibodies of the present disclosure binding to BCMA derived from different species. For example, an antibody of the present disclosure that binds to human BCMA can also bind to BCMA of another species. Cross-reactivity is measured in binding assays (for example SPR and ELISA) by detecting specific reactivity with purified antigen, or the binding or functional interaction with cells that physiologically express BCMA. Methods for determining cross-reactivity include standard binding assays as described herein, for example surface plasmon resonance (SPR) analysis or flow cytometry.

The terms "inhibition" or "blocking" can be used interchangeably and encompass both partial and complete inhibition/blocking. The inhibition/blocking of a ligand preferably reduces or alters the normal level or type of activity that occurs when ligand binding occurs without inhibition or blocking. Inhibition and blocking are also intended to include any measurable reduction in binding affinity of the ligand when contacting with anti-BCMA antibody, compared to the ligand not contacting with anti-BCMA antibody.

The term "inhibition of growth" (for example, of cells) is intended to include any measurable reduction in cell growth.

The terms "induced immune response" and "enhanced immune response" can be used interchangeably and refer to the immune response stimulated (i.e. passive or adaptive) by a specific antigen. The term "induce" for inducing CDC or ADCC refers to stimulating a specific direct cell killing mechanism.

The "ADCC" in the present disclosure, i.e. antibody-dependent cell-mediated cytotoxicity, means that cells expressing Fc receptors directly kill the target cells coated with antibodies by recognizing the Fc segment of the antibodies. The ADCC function of antibodies can be enhanced, reduced or eliminated by modifying the Fc segment of IgG. The modification refers to mutation in the heavy chain constant region of an antibody.

The methods for producing and purifying antibodies and antigen-binding fragments are well-known and can be found in the prior art, such as Antibodies: A Laboratory Manual, Cold Spring Harbor, chapters 5-8 and 15. For example, mice can be immunized with human BCMA or fragment thereof, and the obtained antibodies can be renatured and purified, and subjected to amino acid sequencing by using conventional methods. Antigen-binding fragments can also be prepared by using conventional methods. The antibody or antigen-binding fragment of the present invention is genetically engineered to add one or more human FR region(s) to the non-human CDR regions. The human FR germline sequences can be obtained from the ImmunoGeneTics (IMGT) website http://imgt.cines.fr, or from The Immunoglobulin FactsBook, 2001ISBN012441351.

The engineered antibodies or antigen-binding fragments of the present disclosure can be prepared and purified by conventional methods. The cDNA sequences of the corresponding antibodies can be cloned and recombined into

GS expression vectors. The recombinant immunoglobulin expression vectors can stably transfect CHO cells. As a more recommended prior art, mammalian expression systems can lead to glycosylation of antibodies, especially at the highly conserved N-terminus of the Fc region. Stable clones are obtained by expressing antibodies that specifically bind to human antigens. Positive clones are expanded in serum-free medium of bioreactors to produce the antibodies. The culture fluid into which the antibodies are secreted can be purified and collected by conventional techniques. The antibodies can be filtered and concentrated by conventional methods. Soluble mixtures and multimers can also be removed by conventional methods, for example molecular sieves and ion exchange. The resulting product needs to be frozen immediately, for example at -70°C, or lyophilized.

The antibody of the present disclosure refers to monoclonal antibody. The monoclonal antibody (mAb) in the present disclosure refers to an antibody obtained from a single cloned cell strain, which is not limited to a eukaryotic, prokaryotic or phage cloned cell strain. Monoclonal antibodies or antigen-binding fragments can be obtained by recombination using, for example, hybridoma technology, recombination technology, phage display technology, synthetic technology (such as CDR-grafting) or other existing technologies.

"Administering", "giving" and "treating", when applied to animals, humans, experimental subjects, cells, tissues, organs or biological fluids, refer to contacting the exogenous medicament, therapeutic agent, diagnostic agent or composition with the animals, humans, subjects, cells, tissues, organs or biological fluids. "Administering", "giving" and "treating" can refer to for example treatment, pharmacokinetics, diagnostic, research and experimental methods. Treating cells includes contacting reagents with the cells, and contacting reagents with fluids, wherein the fluids are in contact with the cells. "Administering", "giving" and "treating" also refer to treating for example cells with reagents, diagnostic agents, binding compositions or with another cell in vitro and ex vivo. "Treating", when applied to human, veterinary or research subjects, refers to therapeutic, preventive or prophylactic measures, research and diagnostic applications.

"Treatment" refers to giving an internal or external therapeutic agent, for example comprising any one of the antibodies of the present disclosure, to a patient having one or more disease symptom(s) on which the therapeutic agent is known to have therapeutic effect. Generally, the therapeutic agent is given in an amount effective to alleviate one or more disease symptom(s) in the treated subject or population, either to induce the regression of such symptoms or to inhibit the development of such symptoms to any clinically measurable extent. The amount of therapeutic agent that is effective to alleviate any specific disease symptom (also referred to as a "therapeutically effective amount") can vary according to a variety of factors, for example the disease state, age and body weight of the patient, and the ability of the drug to produce the desired therapeutic effect in the patient. Whether the disease symptoms have been alleviated can be evaluated by any clinical testing methods commonly used by doctors or other health care professionals for evaluating the severity or progression of the symptoms. Although the embodiments of the present disclosure (for example treatment methods or products) may be ineffective in alleviating each disease symptom of interest, but they should reduce the disease symptom of interest in a statistically significant number of patients, as determined by any statistical testing methods known in the art, such as Student t-test, chi-square test, Mann and Whitney’s U test, Kruskal-Wallis test (H test), Jonckheere-Terpstra test and Wilcoxon test.

The term "essentially consisting of......" or variant thereof used throughout the specification and claims means to comprise all the elements or element groups described, and optionally comprise other elements similar or different in nature to the elements described, which does not significantly change the basic or new properties of the given dosing regimen, method or composition.

The term "naturally occurring" applied to a certain object in the present disclosure refers to the fact that the object can be found in nature. For example, a polypeptide sequence or polynucleotide sequence that exists in organisms (including viruses) that can be isolated from natural sources and has not been intentionally modified artificially in the laboratory is naturally occurring.

The "effective amount" includes an amount sufficient to ameliorate or prevent a symptom or condition of a medical condition. The effective amount also refers to an amount sufficient to allow or facilitate diagnosis. The effective amount for a particular patient or veterinary subject can vary depending on the following factors: such as the condition to be treated, the general health condition of the patient, the method, route and dose of drug administration, and the severity of side effects. The effective amount can be the maximum dose or dosing regimen that avoids significant side effects or toxic effects.

"Exogenous" refers to substances produced outside organisms, cells or human bodies depending on backgrounds.

"Endogenous" refers to substances produced inside cells, organisms or human bodies depending on backgrounds.

"Identity" refers to the sequence similarity between two polynucleotide sequences or between two polypeptides. When the positions in the two sequences aligned are occupied by the same base or amino acid monomer subunit, for example if each position of two DNA molecules is occupied by adenine, then the molecules are homologous at that position. The identity percentage between two sequences is a function of the number of matching or homologous positions shared by the two sequences divided by the number of positions to be compared×100%. For example, in the optimal

sequence alignment, if 6 out of 10 positions in the two sequences are matched or homologous, then the two sequences are 60% homologous. Generally, the alignment is made when two sequences are aligned to obtain the maximum identity percentage.

The expressions "cell", "cell line" and "cell culture" used herein can be used interchangeably, and all such names include progeny thereof. Therefore, the words "transformant" and "transformed cell" include primary test cells and cultures derived therefrom, regardless of the number of passages. It should also be understood that due to intentional or unintentional mutations, all offspring cannot be exactly the same in terms of DNA content. Mutant progeny with the same function or biological activity as screened in the original transformed cells is included. It can be clearly seen from the context when different names are referred to.

"Optional" or "optionally" means that the event or circumstance described subsequently can but not necessarily occur, and the description includes the cases where the event or circumstance does or does not occur. For example, "optionally comprising 1 to 3 antibody heavy chain variable regions" means that the antibody heavy chain variable regions of particular sequences can but need not be present.

The "pharmaceutical composition" means comprising one or more of the antibody (antibodies) or antigen-binding fragment(s) described herein as well as other components such as physiological/pharmaceutically acceptable carriers and excipients. The objective of the pharmaceutical composition is to facilitate administration of a compound to an organism, which is conducive to the absorption of the active ingredient so as to show the biological activity.

The term " pharmaceutical salt" refers to a salt of the antibody drug conjugates of the present disclosure. Such salts are safe and effective when used in mammalians and possess the required biological activity. The ligand drug conjugate of the present disclosure at least comprises one amino group and thus may form a salt with an acid. Non-limiting examples of pharmaceutically acceptable salts include: hydrochloride, hydrobromide, hydriodate, sulphate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate and p-toluenesulfonate.

The term "solvate" refers to a pharmaceutically acceptable solvate formed by the antibody-drug conjugate compound of the present disclosure and one or more solvent molecule(s). Non-limiting examples of solvent molecules include water, ethanol, acetonitrile, isopropanol, ethyl acetate.

The term "cytotoxic drug", when used in the present disclosure, refers to a substance that inhibits the function of cells and/or causes cell death or destruction.

The "tubulin inhibitor" refers to a class of compounds that interfere with the process of cell mitosis by inhibiting the polymerization of tubulin or promoting the aggregation of tubulin, thereby exerting an anti-tumor effect. Non-limiting examples thereof include: maytansinoids, calicheamicin, taxanes, vincristine, colchicine, dolastatin/auristatin/monomethyl auristatin E (MMAE)/ monomethyl auristatin F (MMAF).

"Linker" refers to a chemical moiety by which an antibody is covalently attached to a covalent bond or atomic chain of a drug. Non-limiting examples of linkers include: arylene, heteroarylene, PEG, polymethyleneoxy, succinate, succinamide, diglycolate, malonate and caproamide.

"Drug load" (DAR) is represented by y, which is the average number of cytotoxic drugs per antibody in formula (A). The range of drug load in the present invention can be 1-20 cytotoxic drugs (D) per antibody. The antibody-drug conjugate of general formula (A) is a collection of antibodies conjugated to a certain range (1-20) of cytotoxic drugs. The drug load (DAR) in an antibody-drug conjugate from coupling reaction can be characterized by conventional means, for example mass spectrometry, HPLC and ELISA. By these means, the quantitative distribution of the antibody-drug conjugate on the y value can be determined.

The present disclosure also comprises the compounds of formula (I) in various deuterated forms. Each of the available hydrogen atoms attached to the carbon atom can be independently replaced by a deuterium atom. Those skilled in the art can synthesize a compound of formula (I) in a deuterated form with reference to the relevant literatures. The compound of formula (I) in deuterated form can be prepared by employing commercially available deuterated raw materials, or they can be synthesized by conventional techniques with deuterated reagents including, but not limited to, deuterated borane, trideuterated borane in tetrahydrofuran, deuterated lithium aluminum hydride, deuterated iodoethane, deuterated iodomethane and the like.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity. The preparation of conventional pharmaceutical compositions can be found in the Chinese Pharmacopoeia.

The term "pharmaceutically acceptable salt" or "pharmaceutical salt" refers to a salt of the ligand-drug conjugate of the present disclosure or a salt of the compound of the present disclosure, which is safe and effective in mammals and has the desired biological activity. The ligand-drug conjugate of the present disclosure contains at least one amino,

so it can form a salt with an acid. Non-limiting examples of pharmaceutically acceptable salts include hydrochloride, hydrobromide, hydroiodide, sulfate, bisulfate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, methanesulfonate, ethanesulfonate, benzenesulfonate, p-toluenesulfonate.

The term "drug loading" refers to the average number of cytotoxic drugs loaded on each antibody or antigen-binding fragment thereof in the compound of formula (V), and can also be expressed as the ratio of the number of drug to the number of antibody. The drug loading can range from 0 to 12, preferably from 1 to 10 cytotoxic drugs (D) per ligand. In an embodiment of the present invention, the drug loading is expressed as y, also known as DAR value, and exemplary values can be an average of 1, 2, 3, 4, 5, 6, 7, 8, 9, 10. The average number of drugs per ADC molecule after coupling reaction can be determined by conventional identification methods such as UV/visible spectroscopy, mass spectrometry, ELISA test and HPLC.

In an embodiment of the present invention, the cytotoxic drug is conjugated to the N-terminal amino and/or the ε-amino of lysine residues of the ligand via a linking unit. In an embodiment of the present invention, the cytotoxic drug is conjugated to the sulfhydryl group of the ligand via a linking unit. Typically, the number of drug molecules conjugated to the antibody in a coupling reaction will be less than the theoretical maximum.

The following non-limiting methods can be used to control the loading of the ligand-cytotoxic drug conjugates:

(1) controlling the molar ratio of the linking reagent to the monoclonal antibody,
(2) controlling the reaction time and temperature,
(3) selecting different reaction reagents.

The antibody drug conjugate or pharmaceutical salt or solvent thereof in the present disclosure has significant anti-tumor effects and good safety.

## Examples

The examples are incorporated below for further description of the present invention, but these examples do not limit the scope of the present invention. The experimental methods with unspecified conditions in the examples of the present invention generally follow conventional conditions, such as those in Antibodies: A Laboratory Manual and Molecular Cloning: A Laboratory Manual, Cold Spring Harbor; or according to the conditions recommended by the raw material or product manufacturer.

The experimental methods in the examples of the present disclosure for which the specific conditions are not indicated were carried out according to conventional conditions or the conditions recommended by the material or product manufacturers. The reagents for which the specific sources are not indicated are conventional reagents purchased from market.

The known raw materials of the present disclosure can be synthesized by or in accordance with the methods known in the art, or can be purchased from Jiangsu Aikon etc.. As shown in the following table:

| Product name | Company | Item No: |
|---|---|---|
| DBU | Energy chemical | HA090231 |
| DCM | LianYu | 2020092301 |
| Compound d | Jiangsu Aikon | AK20-58300 |
| HATU | Bide | ARC064 |

### Example 1-1: The synthesis of compound a

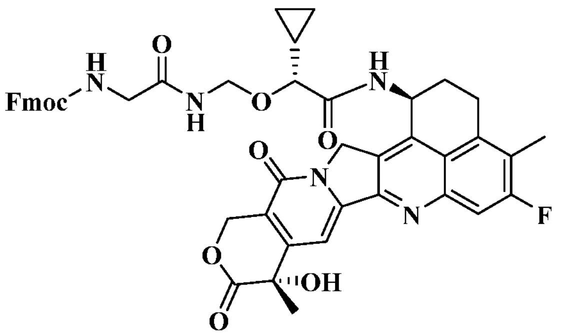

a

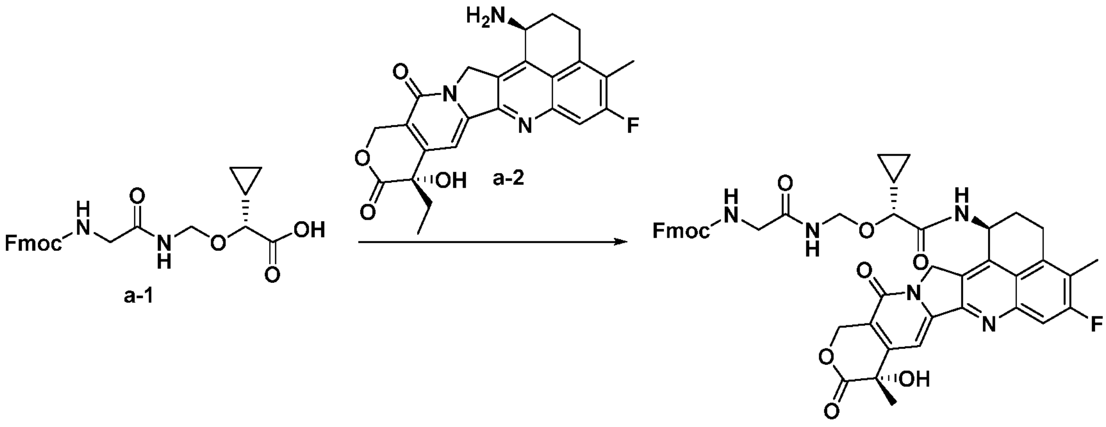

a-2

a-1

a

Raw material a-1 (4.1g, 9.71mmol) and raw material a-2 (4.3 g, 8.09mmol, containing 4% amino isomer impurity) were placed in a 250 mL reaction bottle. DCM (54 mL) and MeOH (18 mL) were added under nitrogen protection, stirred and cooled to 0°C. DMTMM (3.6g, 12.1mmol) and triethylamine (2.5g, 24.2mmol) were added, and stirring reaction was maintained at 0°C for 1h. HPLC central control showed that the material a-2 was completely reacted, and the reaction liquid was dried by reducing pressure (<25°C). MTBE (120mL) was added to stir beating (sludge). The solution was poured out and filtered, 120 mL MTBE was added to the sludge again for beating (solid), filtered, washed the filter cake with water (60 mL×2), and dried to obtain the coarse product, and the coarse product was dissolved in dichloromethane and methanol. Compound a (6.2g, 7.37 mmol) was isolated by running two rounds of wet column chromatography (eluent DCM:MeOH=40:1-20:1) with purity of 99.3% and yield of 91%.

**Example 1-2: The synthesis of compound b**

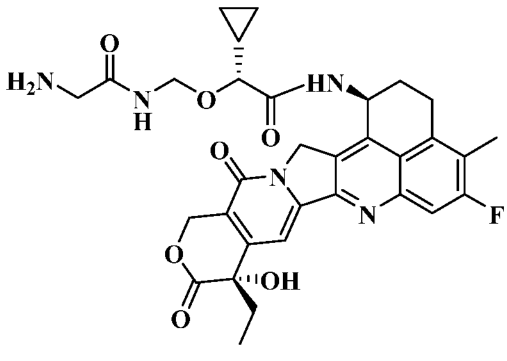

b

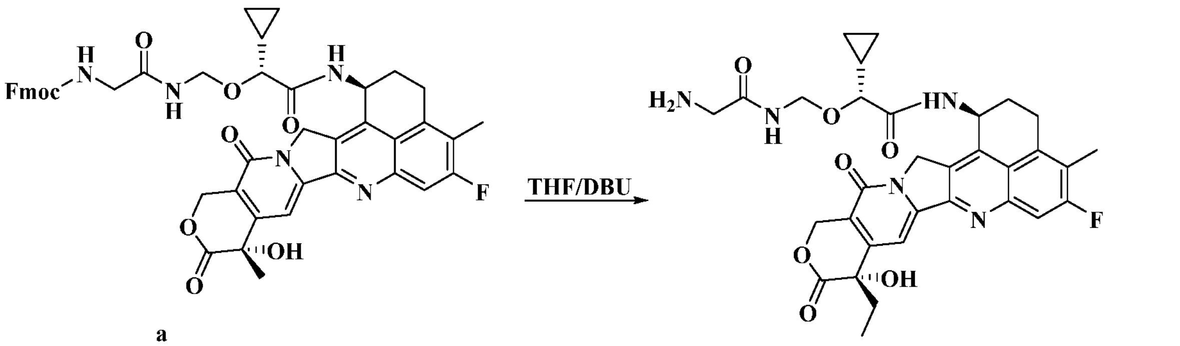


a

b

Compound a (5.7g, 6.77mmol) was placed into a 500ml three necked bottle, dry THF (114ml) was introduced under the protection of nitrogen, stirred to dissolve and cooled the internal temperature to about -10 °C. DBU (3.09g, 20.31mmol) was added by dripping for 5min, the internal temperature was maintained at -10 to -5 °C during dripping. After dripping, the internal temperature was maintained at -10 to -5 °C, the reaction lasted for 2.5h, and the solid precipitated.

The internal temperature was cooled to -20°C, MTBE (114 mL) was added, and the internal temperature was maintained at -20 to -10°C during the period. The product was completely precipitated and filtered, and the filter cake was washed with MTBE (57 mL×2). After being drained, 6 g crude compound b was obtained and stored at -78°C for later use.

**Example 1-3: The synthesis of compound e**

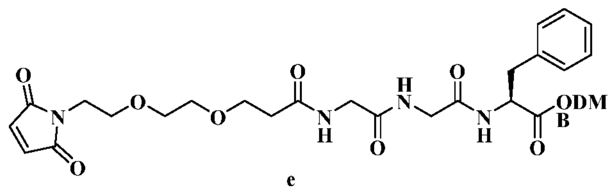

e

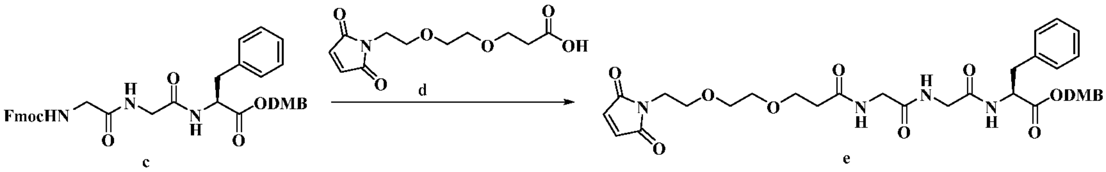


c

e

Compound c (651mg, 1mmol) was dissolved in 10mL DCM, stirring was started in the ice bath, and DBU (456mg, 3mmol) was added by dripping. The reaction was completed after one hour in the ice bath, and the compounds d (257mg, 1mmol) and HATU (420mg, 1.1mmol) were added sequentially. Stirring was performed in ice bath for 30 minutes, LCMS showed that the reaction was complete. The reaction solution was concentrated at 25°C, and the residue was purified by column machine (ACN in $H_2O$, 50% of the product) to obtain compound e, reddish brown solid, 130mg,

with a yield of 19%.

MS: 691.3 [M+23].

**Example 1-4: The synthesis of compound f**

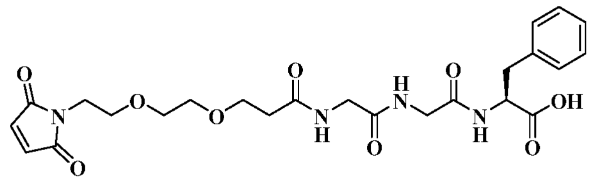

f

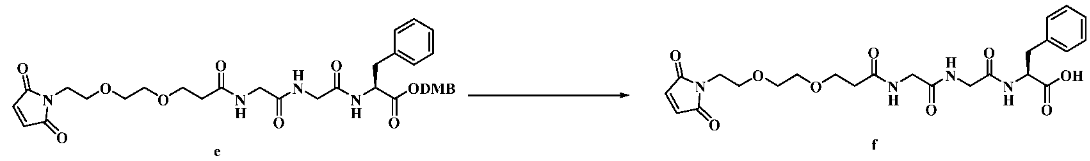

e

f

Compound e (130mg, 0.19mmol) was dissolved in DCM, anisole (62mg, 0.57mmol) and dichloroacetic acid (245mg, 1.9mmol) were added, and the reaction was stirred at room temperature overnight for 16 hours. Sampling, LC-MS central control showed that raw materials were completely consumed, and the reaction was stopped. The reaction solution was concentrated at 25°C, and the residue was purified by the column machine of reverse phase column (ACN/$H_2O$, 30% of the product) to obtain compound f, pink solid, 53mg, with a yield of 54%.

MS: 519.2[M+1].

**Example 1-5: The synthesis of compound d**

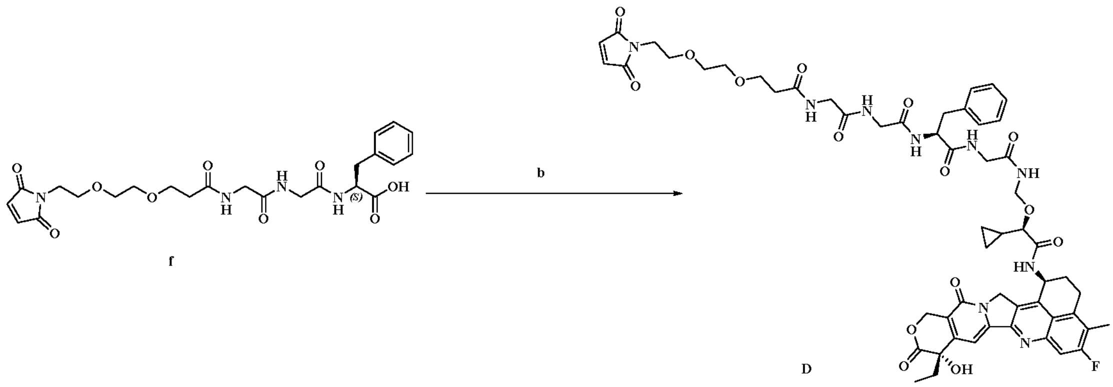


f

D

Compound f (23mg, 0.044mmol) and compound b (27mg, 0.044mmol) were dissolved in DCM (3mL) and MeOH (1mL) and cooled to -30°C under nitrogen protection. DMTMM (20mg, 0.067mmol) was added, and the reaction was performed at a controlled temperature of -20°C to -10°C for 1 hour. Sampling, LC-MS central control showed that the raw material was completely consumed. The temperature was maintained at -10°C, 10mL water was added to quench the reaction , 30mL DCM was added for separation by stratification . The aqueous phase was extracted with DCM/MeOH = 10/1 (50mL). The organic phase was pooled, dried with anhydrous sodium sulfate and concentrated under reduced pressure at 25°C. The residue was purified (ACN/$H_2O$/ 0.05% FA) to obtain compound d, 5.8 mg white solid with yield of 12% and purity of 98.87% by HPLC.

MS: 1120.3[M+1].

**Example 2: Preparation of antigens**

The protein encoding the His-tagged extracellular domain of human BCMA (BCMA-His) was synthesized by

SinoBiologics (Cat No.: 10620-H08H).

Sequence of BCMA-His:

MLQMAGQCSQNEYFDSLLHACIPCQLRCSSNTPPLTCQRYCNASVTNSVKGT
NAHHHHHHHHHH

SEQ ID NO: 21.

**Example 3: Obtaining murine hybridomas and antibody sequences**

A total of 5 Balb/c and 5 A/J female 10-week-old mice were immunized with the human antigen BCMA-His. Sigma Complete Freund's Adjuvant (CFA) and Sigma Incomplete Freund's Adjuvant (IFA) were used. The immunogen and the immune adjuvant were thoroughly mixed at a ratio of 1:1 and emulsified to make a stable "water-in-oil" liquid. The injection dose was 25 μg/200 μL/mouse.

Table 1. Immunization scheme

| | |
|---|---|
| Day 01 | First immunization, complete Freund's adjuvant |
| Day 21 | Second immunization, incomplete Freund's adjuvant |
| Day 35 | Third immunization, incomplete Freund's adjuvant |
| Day 42 | Blood sampling and serum titer test (blood after 3 immunizations) |
| Day 49 | Fourth immunization, incomplete Freund's adjuvant |
| Day 56 | Blood sampling and serum titer test (blood after 4 immunizations) |

The serum titer and the ability to bind to cell surface antigens of the serum of immunized mice were evaluated by using indirect ELISA method according to Example 3. The start of cell fusion was determined according to the detection results of titer (greater than 100,000 times of dilution). The immunized mice with high serum titer, affinity and FACS binding were selected for one final immunization and then sacrificed. The spleen cells were fused to SP2/0 myeloma cells and plated to obtain hybridomas. The hybridomas of interest were screened by indirect ELISA and established as monoclonal cell strains by limiting dilution method. The resulting antibody-positive strains were further screened by indirect ELISA so as to select the hybridomas that bind to the recombinant protein. Hybridoma cells at logarithmic growth phase were collected. RNA was extracted with Trizol (Invitrogen, 15596-018) and subjected to reverse transcription (PrimeScript ™ Reverse Transcriptase, Takara #2680A). The cDNA obtained by reverse transcription was amplified by PCR using a mouse Ig-primer set (Novagen, TB326 Rev.B 0503). Finally, the sequence of murine antibody M1 was obtained.

The heavy chain and light chain variable region sequences of murine monoclonal antibody M1 are as follows:

M1 HCVR

QVQLQQSGAELVRPGASVKLSCKALGYSFSDYEMHWVRQTPVHGLEWIGGI
HPGSGGSAYNQKFKGKATLTVDKSSSTAYMELSSLTSEDSAVYYCTRLDYGYS
WAWFPYWGQGTLVTVSA

SEQ ID NO: 1

M1 LCVR

EILLTQSPAIIVTSPGEKVTITCSASSSVIYMNWYQQKPGSSPKIWIYGISNLASG
VPARFSGSGSGTSFSFTINSMEAEDVATYYCQQRSSYPLTFGAGTKLELK

SEQ ID NO: 2

Table 2. The CDR sequences of the heavy chain and light chain variable regions of murine monoclonal antibody M1

| Name | Sequence | NO. |
|---|---|---|
| HCDR1 | GYSFSDYEMH | SEQ ID NO: 3 |
| HCDR2 | GIHPGSGGSAYNQKFKG | SEQ ID NO: 4 |
| HCDR3 | TRLDYGYSWAWFPY | SEQ ID NO: 5 |
| LCDR1 | SASSSVIYMN | SEQ ID NO: 6 |
| LCDR2 | GISNLAS | SEQ ID NO: 7 |
| LCDR3 | QQRSSYPLT | SEQ ID NO: 8 |

**Examples 4: Detection method for in vitro binding activity of antibodies**

(1) In vitro indirect ELISA binding assay:

BCMA His protein (Sino Biological Inc., cat# 10620-H08H) was diluted with pH 7.4 PBS to a concentration of 1 μg/ml, added to a 96-well high-affinity ELISA plate at 100μl/well and incubated in a refrigerator at 4°C overnight (16-20 hours). After washing the plate 4 times with PBST (pH 7.4 PBS containing 0.05% Tween-20), 3% bovine serum albumin (BSA) blocking solution diluted with PBST was added at 150 μl/well and incubated at room temperature for 1 hour for blocking. After completion of the blocking, the blocking solution was discarded, and the plate was washed 4 times with PBST buffer.

The antibody to be tested was 10-fold gradient diluted with PBST containing 3% BSA with the initial concentration of 1μM to obtain 10 dilutions which were added to the microtiter plate at 100μl/well and incubated at room temperature for 1 hour. After completion of the incubation, the plate was washed 4 times with PBST. HRP-labeled goat-anti-human secondary antibody (Abeam, cat#ab97225) diluted with PBST containing 3% BSA was added at 100μl/well and incubated at room temperature for 1 hour. The plate was washed 4 times with PBST, and then TMB chromogenic substrate (Cell Signaling Technology, cat#7004S) was added at 100 μl/well and incubated at room temperature in the dark for 1 minute. The stop solution (Cell Signaling Technology, cat#7002S) was added at 100μl/well to terminate the reaction. The absorbance value at 450nm was read with a microplate reader (BioTek, model Synergy H1). The data were analyzed. The results were analyzed by plotting the concentration-signal curve, as shown in the following table:

Table 3. Affinity of murine antibodies to human BCMA antigen ($EC_{50}$ value

| Murine antibody | $EC_{50}$ (nM) of the binding to human BCMA His antigen |
|---|---|
| M1 | 0.53 |

*(2) In vitro* cell binding assay:

The cultured cells with high BCMA expression (HEK-293T cells overexpressing BCMA; tumor cells expressing BCMA, NCI-H929) were collected. The cell density was adjusted and the cells were plated on a 96-well U-bottomed plate at $1\times10^5$ to $2\times10^5$ cells per well. The plate was centrifuged at 1200g for 5 min and the supernatant was removed. 100μl of gradient diluted antibody solution or mouse immunized serum was added and incubated at 4°C for 60 min. The plate was centrifuged at 1200g for 5 min and the supernatant was removed. The cells were washed twice with PBS. A fluorescently labeled secondary antibody (PE-GAH or PE-GAM) was added at 100μl per well and incubated at 4°C for 60 min. The plate was centrifuged at 1200g for 5 min and the supernatant was removed. The cells were washed twice with PBS and then re-suspended in PBS. The signal was detected by using a flow cytometer, and a concentration curve was plotted for result analysis.

Table 4. Affinity of murine antibodies to cells expressing BCMA ($EC_{50}$ value

| Murine antibody | $EC_{50}$ (nM) of the binding to HEK-293T/BCMA cells | $EC_{50}$ (nM) of the binding to NCI-H929 cells |
|---|---|---|
| M1 | 115.3 | 128.2 |

**Example 5: Humanization experiment of murine antibodies**

Humanization of the murine anti-human BCMA monoclonal antibodies was performed according to methods as published in many documents in the art. Briefly, parental (murine antibody) constant domains were replaced with human constant domains. Human germline antibody sequences were selected according to the identity between the murine antibody and human antibody. The murine antibody M1 was humanized in the present disclosure.

On the basis of the typical structure of the VH/VL CDR of the obtained murine antibody, the sequences of heavy and light chain variable regions were aligned with the human antibody germline database to obtain human germline templates with high identity.

The CDR regions of the murine antibody M1 were grafted to the selected corresponding humanization templates. Then, based on the three-dimensional structure of the murine antibody, the embedded residues, the residues directly interacting with the CDR regions and the residues with significant influence on the conformation of VL and VH were subjected to back mutation, and chemically unstable amino acid residues in the CDR regions were optimized. After expression testing and comparison of the number of back mutations, the sequences of the humanized heavy chain variable region HCVRs were selected and designed, which are as follows:

HCVR1

QVQLVQSGAEVKKPGASVKVSCKASGYSFSDYEMHWVRQAPGQGLEWMGG
IHPGSGGSAYNQKFKGRVTMTRDTSTSTVYMELSSLRSEDTAVYYCTRLDYG
YSWAWFPYWGQGTLVTVSA

SEQ ID NO: 9

HCVR2

QVQLVQSGAEVKKPGASVKVSCKASGYSFSDYEMHWVRQAPGQGLEWIGGI
HPGSGGSAYNQKFKGRVTLTVDKSTSTAYMELSSLRSEDTAVYYCTRLDYGYS
WAWFPYWGQGTLVTVSA

SEQ ID NO: 10

HCVR3

QVQLVQSGAEVKKPGASVKVSCKASGYSFSDYEMHWVRQAPGQGLEWIGGI
HPGSGGSAYNQKFKGKATLTVDKSTSTAYMELSSLRSEDTAVYYCTRLDYGYS
WAWFPYWGQGTLVTVSA

SEQ ID NO: 11

The sequences of the humanized light chain variable region LCVRs were selected and designed, which are as follows:

LCVR1

EIVLTQSPATLSLSPGERATLSCSASSSVIYMNWYQQKPGQAPRLLIYGISNLAS
GIPARFSGSGSGTDFTLTISSLEPEDFAVYYCQQRSSYPLTFGGGTKVEIK

SEQ ID NO: 12

LCVR2

EIVLTQSPATLSLSPGERATLSCSASSSVIYMNWYQQKPGQSPKIWIYGISNLAS
GVPARFSGSGSGTDFTLTISSLEPEDFAVYYCQQRSSYPLTFGGGTKVEIK

SEQ ID NO: 13

LCVR3

EILLTQSPATLSLSPGERATLTCSASSSVIYMNWYQQKPGSSPKIWIYGISNLAS
GVPARFSGSGSGTSFTLTISSLEPEDFAVYYCQQRSSYPLTFGGGTKVEIK

SEQ ID NO: 14

The designed heavy chain and light chain variable region sequences were linked to the human IgG1 heavy chain and light chain constant region sequences, respectively. Exemplary heavy chain and light chain constant region sequences are respectively as follows:

IgG1 C

ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALP
APIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNH
YTQKSLSLSPGK

SEQ ID NO: 22

Ig kappa C

RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C

SEQ ID NO: 23

Exemplary heavy chain and light chain sequences obtained are as follows:

Ab1 HC

QVQLVQSGAEVKKPGASVKVSCKASGYSFSDYEMHWVRQAPGQGLEWMGG
IHPGSGGSAYNQKFKGRVTMTRDTSTSTVYMELSSLRSEDTAVYYCTRLDYG
YSWAWFPYWGQGTLVTVSAASTKGPSVFPLAPSSKSTSGGTAALGCLVKDYF
PEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPE
VTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKS
RWQQGNVFSCSVMHEALHNHYTQKSLSLSPGK

SEQ ID NO：15

Ab2 HC

QVQLVQSGAEVKKPGASVKVSCKASGYSFSDYEMHWVRQAPGQGLEWIGGI
HPGSGGSAYNQKFKGRVTLTVDKSTSTAYMELSSLRSEDTAVYYCTRLDYGYS
WAWFPYWGQGTLVTVSAASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW
QQGNVFSCSVMHEALHNHYTQKSLSLSPGK

SEQ ID NO：16

Ab3 HC

QVQLVQSGAEVKKPGASVKVSCKASGYSFSDYEMHWVRQAPGQGLEWIGGI
HPGSGGSAYNQKFKGKATLTVDKSTSTAYMELSSLRSEDTAVYYCTRLDYGYS
WAWFPYWGQGTLVTVSAASTKGPSVFPLAPSSKSTSGGTAALGCLVKDYFPE
PVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRW
QQGNVFSCSVMHEALHNHYTQKSLSLSPGK

SEQ ID NO: 17

Ab1 LC

EIVLTQSPATLSLSPGERATLSCSASSSVIYMNWYQQKPGQAPRLLIYGISNLAS
GIPARFSGSGSGTDFTLTISSLEPEDFAVYYCQQRSSYPLTFGGGTKVEIKRTVA
APSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 18

Ab2 LC

EIVLTQSPATLSLSPGERATLSCSASSSVIYMNWYQQKPGQSPKIWIYGISNLAS
GVPARFSGSGSGTDFTLTISSLEPEDFAVYYCQQRSSYPLTFGGGTKVEIKRTVA
APSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 19

Ab3 LC

EILLTQSPATLSLSPGERATLTCSASSSVIYMNWYQQKPGSSPKIWIYGISNLAS
GVPARFSGSGSGTSFTLTISSLEPEDFAVYYCQQRSSYPLTFGGGTKVEIKRTVA
APSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 20

Table 5. Antibodies and SEQ ID NOs. of heavy chain, light chain and variable regions thereof

| Humanized antibody NO. | Heavy chain/light chain NO. | Heavy chain/light chain variable region NO. |
|---|---|---|
| Ab1 | SEQ ID NO: 15 | SEQ ID NO: 9 |
| | SEQ ID NO: 18 | SEQ ID NO: 12 |
| Ab2 | SEQ ID NO: 16 | SEQ ID NO: 10 |
| | SEQ ID NO: 19 | SEQ ID NO: 13 |
| Ab3 | SEQ ID NO: 17 | SEQ ID NO: 11 |
| | SEQ ID NO: 20 | SEQ ID NO: 14 |

cDNA fragments were synthesized according to the amino acid sequences of the light chain and heavy chain of each humanized antibody above, and inserted into the pcDNA3.1 expression vector (Life Technologies Cat. No. V790-20). The expression vector was used along with the transfection reagent PEI (Polysciences, Inc. Cat. No. 23966) at a ratio of 1:2 to transfect HEK293 cells (Life Technologies Cat.No. 11625019). The cells were incubated in a $CO_2$ incubator for 4-5 days. The cell culture fluid was collected, centrifuged and filtered. Then the samples were loaded on the antibody purification affinity column. The column was washed with phosphate buffer. The samples were eluted with glycine-hydrochloric acid buffer (pH 2.7, 0.1M Gly-HCl), neutralized with 1M Tris hydrochloric acid pH 9.0, and dialyzed against phosphate buffer to obtain the humanized antibody proteins of the present disclosure.

**Example 6: *In vitro* binding affinity and kinetic assay**

The affinity ($EC_{50}$) of each humanized antibody to human BCMA antigen was determined by using the in vitro

indirect ELISA binding assay according to Example 4 (1), and is as shown in table below:

Table 6. Affinity of each humanized antibody to human BCMA antigen ($EC_{50}$)

| Humanized antibody | Antigen | Affinity $EC_{50}$ (nM) |
|---|---|---|
| Ab1 | BCMA-His | 0.022 |
| Ab2 | | 0.033 |
| Ab3 | | 0.034 |

The affinity ($EC_{50}$) of each humanized antibody to NCI-H929 tumor cells was determined by using the in vitro cell binding assay according to Example 4 (2), and is as shown in Table 7 below:

Table 7. Affinity ($EC_{50}$) of each humanized antibody to NCI-H929 tumor cells

| Humanized antibody | Cell | Affinity EC50 (nM) |
|---|---|---|
| Ab1 | NCI-H929 | 4.6 |
| Ab2 | | 3.5 |
| Ab3 | | 3.9 |

**Example 7: Endocytosis of the antibodies**

NCI-H929 was used for evaluating whether the antibodies of the present disclosure could be endocytosed into cells along with human BCMA after binding to BCMA. NCI-H929 cells were digested with trypsin (after washed once with PBS at 37°C for about 2 min), collected and re-suspended in pre-cooled FACS buffer. The cell concentration was adjusted to $1\times10^6$ cells/mL. 1mL of cell suspension was added to an EP tube, centrifuged at 1500 rpm for 5 minutes, and the supernatant was removed. 1 mL of the prepared antibody to be tested was added to re-suspend the cells, and the final concentration of the antibody was 20 μg/ml. The cells were incubated in a shaker at 4°C for 1 hour, centrifuged (4°C, 1500 rpm×5 min), and the supernatant was discarded. The cells were washed twice with FACS buffer and the supernatant was removed. 100μL of fluorescent secondary antibody working solution was added to each tube to re-suspend the cells. The cells were incubated in a shaker at 4°C for 30 min, centrifuged (4°C, 1500 rpm×5 min), and the supernatant was discarded. The cells were washed twice with FACS buffer and the supernatant was removed. 1.0mL of pre-warmed NCI-H929 cell complete medium was added to each tube to re-suspend the cells and mixed thoroughly. The cell suspension was aliquoted into 4 tubes, 200μL per tube, respectively as 0 min group, blank group, 30 min group and 2 h group. The 0 min and blank groups were placed on ice, while the rest groups were placed in an incubator at 37°C for endocytosis for 30min and 2h respectively. At the corresponding time point, the EP tube was taken out and placed on ice to pre-cooled for 5min. All treatment groups were centrifuged (4°C, 1500 rpm×5min) and the supernatant was discarded. The cells were washed once with FACS buffer and the supernatant was removed. 250μL strip buffer was added to EP tubes of all treatment groups except the 0 min group. The cells were incubated at room temperature for 8 min, centrifuged (4°C, 1500 rpm×5min) and the supernatant was discarded. The cells were washed twice with FACS buffer and the supernatant was removed. All treatment groups were added with 100μL fixing solution, placed at 4°C for more than 30min for immunostaining, and detected by the flow cytometer DxFlex. Percentage of BCMA antibody endocytosis = (fluorescence intensity value at each time point - average fluorescence intensity value of the 0 min group) / average fluorescence intensity value of the 0 min group. The results are shown in table below:

Table 8. Endocytosis $EC_{50}$ of antibodies into NCI-H929 tumor cells

| Humanized antibody | Cell | Endocytosis efficiency % | |
|---|---|---|---|
| | | 0.5 hour | 2 hours |
| Ab1 | NCI-H929 | 36.6 | 49.5 |
| Ab2 | | 36.7 | 48 |
| Ab3 | | 35.5 | 45.7 |

**Example 8: Preparation of antibody drug conjugates**

Preparation of ADC2:

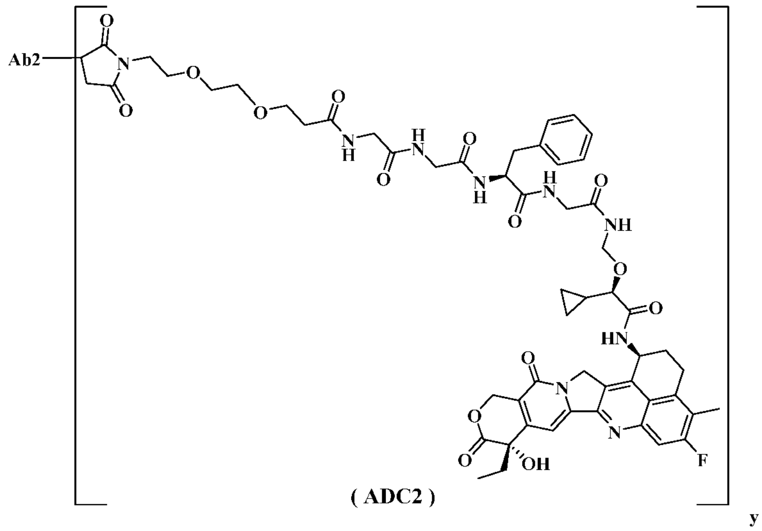

( ADC2 )

A formulated aqueous solution of tris(2-carboxyethyl)phosphine (10 mM, 0.347 mL, 3.47 μmol) was added to a PBS-buffered aqueous solution of antibody Ab2 (0.05 M PBS-buffered aqueous solution with pH=6.5; 7.3 ml, 13.8 mg/ml, 0.681 μmol) at 37°C.The reaction solution was placed in a water bath shaker, and shaked at 37°C for 3 hours before stopping the reaction. The reaction solution was cooled to 25°C in a water bath, and diluted to 14.0 ml. 3.3 ml of the solution was taken for the next reaction.

Compound D (3.0mg, 3.72μmol) was dissolved in 0.15ml DMSO, and then added to the above 3.3ml solution. The reaction solution was placed in a water bath shaker, and shaked at 25°C for 3 hours before stopping the reaction.. The reaction solution was desalted and purified with a Sephadex G25 gel column (elution phase: 0.05M PBS-buffered aqueous solution with pH= 6.5, containing 0.001M EDTA) to obtain the PBS-buffered solution of the exemplary product ADC2 as shown in formula Ab2 antibody conjugate (1.35mg/ml, 13ml). The solution was frozen at 4 °C.

The average value y was determined by the ultraviolet method. Cuvettes filled with sodium succinate buffer were respectively placed in the reference absorption cell and the sample determination absorption cell, and after deducting the solvent blank, the cuvettes filled with the test solution were placed in the sample determination absorption cell. The absorbance at 280nm and 370nm was measured.

Data processing:

The antibody content $C_{Ab}$ was determined by establishing a standard curve and measuring the absorption at the wavelength of 280nm. The small molecule content $C_{Drug}$ was determined by measuring the absorption at the wavelength of 370nm.

$$\text{Average value of drug load } y = C_{Drug}/C_{Ab}.$$

As for the exemplary product ADC2, y was determined to be 4 by the above method. Samples of ADC2 (y=4) were obtained by UV-HPLC purification.

**Example 9: The killing activity of antibody drug conjugates on tumor cells**

In order to test the killing effect of the antibody drug conjugates of the present disclosure on tumor cells, The BCMA high expression cell line NCI-H929 cells (ATCC deposit number CRL-9068) were used for evaluation. NCI-H929 cells were collected, centrifuged and counted. The cell density was adjusted to $1\times10^5$ cells/mL with complete medium, and the cells were plated in 60 wells of a white 96-well plate at 100μL per well with a cell number of 10,000 cells/well. The rest peripheral wells were added with 100μL DPBS per well. The cell plate was placed in a 37°C, 5% $CO_2$ incubator and cultured overnight. On the second day of the experiment, the antibody-drug conjugate working solution was prepared with complete medium in a 96-well V-bottom plate, starting from a concentration of 133 μM (5-fold dilution and 9 concentrations). After completion of the preparation, the solution was added to a white 96-well plate at 80μL per well in duplicates. The cell plate was placed in a 37°C, 5% $CO_2$ incubator and the culture lasted for 72 hours. On the fifth day of the experiment, the plate was detected and read. The cell culture plate was taken out. 90μL CellTiter-Glo® solution (Promega, Cat#: G7573) was added to each well after equilibrating to room temperature. The mixture was shaken and mixed, placed in dark and let stand for 10 minutes, and then detected by using the luminescence program of the microplate reader. The $IC_{50}$ values were calculated using GraphPad Prims software. The ADC (y=4) of negative antibody IgG1 conjugated with compound D was used as negative control. The experimental results are shown in the table below:

Table 9: Evaluation of the killing activity of antibody drug conjugates on tumor cells

| Antibody drug conjugate | Killing Effect ($IC_{50}$ nM) NCI-H929 |
|---|---|
| ADC2 (y=4) | 0.76 |
| IgG1 iso ADC (y=4) | >133 |

The experimental results showed that ADC2 (y=4) had good killing activity on tumor cells *in vitro.*

**Example 10: Tumor killing activity of antibody drug conjugates**

In order to further investigate the inhibition effect of the antibody drug conjugates on the proliferation of tumors formed *in vivo*, the anti-tumor effect of the antibody drug conjugates of the present disclosure was evaluated after formation of transplanted tumors with NCI-H929 (ATCC deposit number CRL-9068) cell line in mice. $10\times10^6$ NCI-H929 cells were injected subcutaneously into the back of 6-8 week-old immunodeficient nude mice (NOD-SCID). After 14 days, intravenous injection of the antibody drug conjugates via caudal vein were performed, at a frequency of once/week, continuous administration for two weeks at a dose of 3 mg/kg. Human IgG1 isotype control protein was used as the control at a dose of 3 mg/kg. There were 5 mice in each group of the control group or the administration group. The tumor inhibition rate was calculated by measuring the tumor volume, as follows:

Tumor inhibition rate TGI% = 100% - (tumor volume of the administration group on day 14 - tumor volume of the administration group on day 0) / (tumor volume of the control group on day 14 - tumor volume of the control group on day 0).

The experimental results are shown in the table below:

Table 10. Killing activity of antibody drug conjugates on tumors

| Group | Tumor inhibition rate (%) |
|---|---|
| IgG1 iso ADC (y=4), 3 mpk | - |
| ADC2 (y=4), 3 mpk | 164.67 |

The experimental results showed that the antibody drug conjugate ADC2 (y=4) showed good tumor inhibition effect *in vivo.*

## Claims

1. An antibody drug conjugate represented by general formula (I) or a pharmaceutically acceptable salt or solvate thereof,

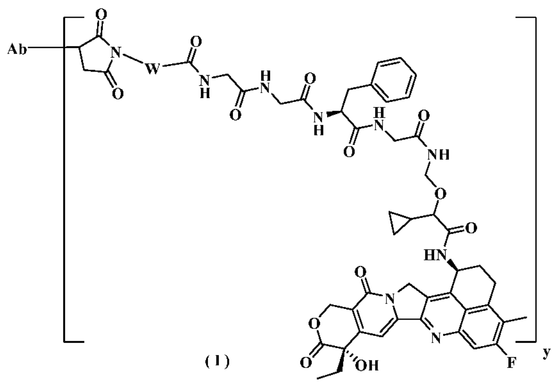

(I)

wherein:

W is $-(CR^eR^f)_g-X_1-(CR^eR^f)_u-X_2-(CR^eR^f)_h-$;
$R^e$ or $R^f$ is independently selected from the group consisting of hydrogen, deuterium, hydroxyl, amino, alkyl, halogen, haloalkyl, deuterated alkyl, hydroxyalkyl; preferably, $R^e$ or $R^f$ is independently selected from the group consisting of hydrogen, deuterium; more preferably, $R^e$ or $R^f$ is hydrogen;
$X_1$ or $X_2$ is independently selected from the group consisting of N, H, O or S; preferably, $X_1$ or $X_2$ is independently selected from S; more preferably, $X_1$ and/or $X_2$ are independently selected from O;
g, u or h is independently selected from the group consisting of 1, 2, 3 and 4; preferably, g, u or h is independently selected from the group consisting of 1, 2, 3; more preferably, g, u or h is 2;
y is 1-20; preferably, y is 4-10; more preferably, y is 4, 6, 8 or 10;
Ab is an anti-BCMA antibody or an antigen-binding fragment thereof, wherein the anti-BCMA antibody or the antigen-binding fragment thereof comprises a heavy chain variable region and a light chain variable region, wherein the heavy chain variable region comprises an HCDR1, an HCDR2 and an HCDR3 set forth in SEQ ID NO: 3, SEQ ID NO: 4 and SEQ ID NO: 5, respectively, and the light chain variable region comprises an LCDR1, an LCDR2 and an LCDR3 set forth in SEQ ID NO: 6, SEQ ID NO: 7 and SEQ ID NO: 8, respectively.

**2.** The antibody drug conjugate or the pharmaceutically acceptable salt or solvate thereof according to claim 1, wherein the anti-BCMA antibody or the antigen-binding fragment thereof is a murine antibody, a chimeric antibody, a human antibody, or a humanized antibody.

**3.** The antibody drug conjugate or the pharmaceutically acceptable salt or solvate thereof according to claim 2, wherein the anti-BCMA antibody or the antigen-binding fragment thereof further comprises a heavy chain constant region derived from human IgG1, IgG2, IgG3 or IgG4 or variant thereof; preferably, the anti-BCMA antibody or the antigen-binding fragment thereof further comprises a heavy chain constant region derived from human IgG1, IgG2, or IgG4 thereof; more preferably, the anti-BCMA antibody or the antigen-binding fragment thereof further comprises IgG1 heavy chain constant region with enhanced ADCC toxicity after amino acid mutation; alternatively, the anti-BCMA antibody or the antigen-binding fragment thereof further comprises heavy chain constant region as shown in SEQ ID NO: 22.

**4.** The antibody drug conjugate or the pharmaceutically acceptable salt or solvate thereof according to claim 2, wherein the anti-BCMA antibody orthe antigen-binding fragment thereof further comprises a light chain constant region derived from human κ chain, λ chain or variant thereof; preferably, the anti-BCMA antibody or the antigen-binding fragment thereof further comprises a light chain constant region derived from human κ chain; more preferably, the anti-BCMA antibody or the antigen-binding fragment thereof further comprises a light chain constant region as shown in SEQ ID NO: 23.

**5.** The antibody drug conjugate or the pharmaceutically acceptable salt or solvate thereof according to claim 2, wherein:

the anti-BCMA antibody or the antigen-binding fragment thereof comprises a heavy chain variable region selected from the group consisting of SEQ ID NO: 9, SEQ ID NO: 10 and SEQ ID NO: 11, or a heavy chain variable region having at least 70%, 75%, 80%, 85%, 90%, 95% or 99% identity with the same.
and/or the anti-BCMA antibody or the antigen-binding fragment thereof comprises a light chain variable region selected from the group consisting of SEQ ID NO: 12, SEQ ID NO: 13 and SEQ ID NO: 14, or a light chain

variable region having at least 70%, 75%, 80%, 85%, 90%, 95% or 99% identity with the same.

**6.** The antibody drug conjugate or the pharmaceutically acceptable salt or solvate thereof according to claim 5, wherein:

the anti-BCMA antibody or the antigen-binding fragment thereof comprises a heavy chain variable region as shown in SEQ ID NO: 9 and a light chain variable region as shown in SEQ ID NO: 12; or
the anti-BCMA antibody or the antigen-binding fragment thereof comprises a heavy chain variable region as shown in SEQ ID NO: 10 and a light chain variable region as shown in SEQ ID NO: 13; or
the anti-BCMA antibody or the antigen-binding fragment thereof comprises a heavy chain variable region as shown in SEQ ID NO: 11 and a light chain variable region as shown in SEQ ID NO: 14.

**7.** The antibody drug conjugate or the pharmaceutically acceptable salt or solvate thereof according to claim 5, wherein:

the anti-BCMA antibody or the antigen-binding fragment thereof comprises a heavy chain as shown in SEQ ID NO: 15, SEQ ID NO: 16 or SEQ ID NO: 17, or a heavy chain having at least 80%, 85%, 90%, 95% or 99% identity with the same;
and/or the anti-BCMA antibody or the antigen-binding fragment thereof comprises a light chain as shown in SEQ ID NO: 18, SEQ ID NO: 19 or SEQ ID NO: 20, or a light chain having at least 80%, 85%, 90%, 95% or 99% identity with the same.

**8.** The antibody drug conjugate or the pharmaceutically acceptable salt or solvate thereof according to claim 7, wherein:

the anti-BCMA antibody comprises a heavy chain as shown in SEQ ID NO: 15 and a light chain as shown in SEQ ID NO: 18, or
the anti-BCMA antibody comprises a heavy chain as shown in SEQ ID NO: 16 and a light chain as shown in SEQ ID NO: 19, or
the anti-BCMA antibody comprises a heavy chain as shown in SEQ ID NO: 17 and a light chain as shown in SEQ ID NO: 20.

**9.** The antibody drug conjugate or the pharmaceutically acceptable salt or solvate thereof according to any one of claims 1 to 8, wherein the antibody drug conjugate or the pharmaceutically acceptable salt or solvate is as shown in general formula (II), or a tautomer, mesomer, racemate, enantiomer, diastereomer or mixture form thereof;

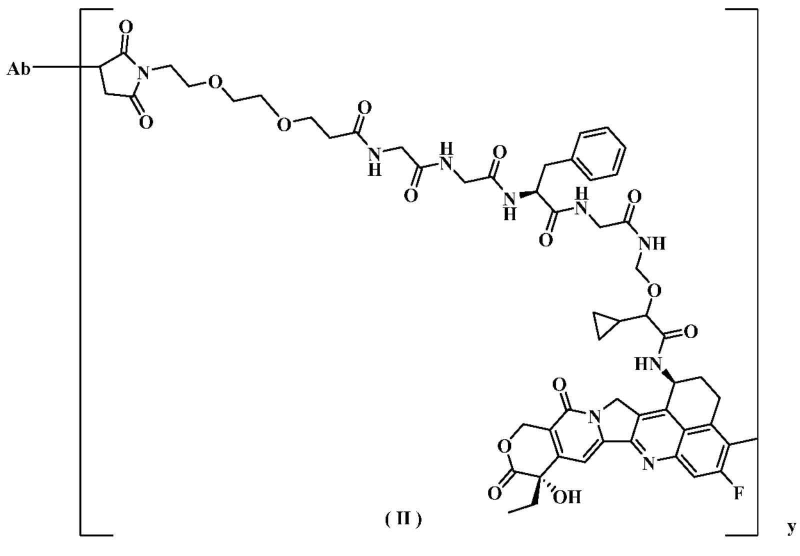

( II )

wherein Ab and y are as defined in claim 1.

**10.** The antibody drug conjugate or the pharmaceutically acceptable salt or solvate thereof according to claims 9, wherein the antibody drug conjugate or the pharmaceutically acceptable salt or solvate is as shown in general formula (III);

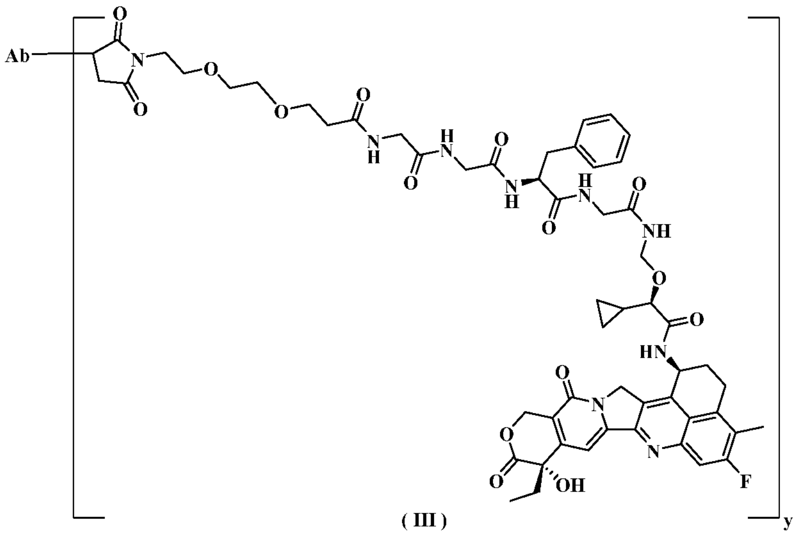

( III )

wherein Ab and y are as defined in claim 1.

**11.** The antibody drug conjugate or the pharmaceutically acceptable salt or solvate thereof according to any one of claims 1 to 10, wherein the antibody drug conjugate or the pharmaceutically acceptable salt or solvate is selected from the group consisting of the following structures:

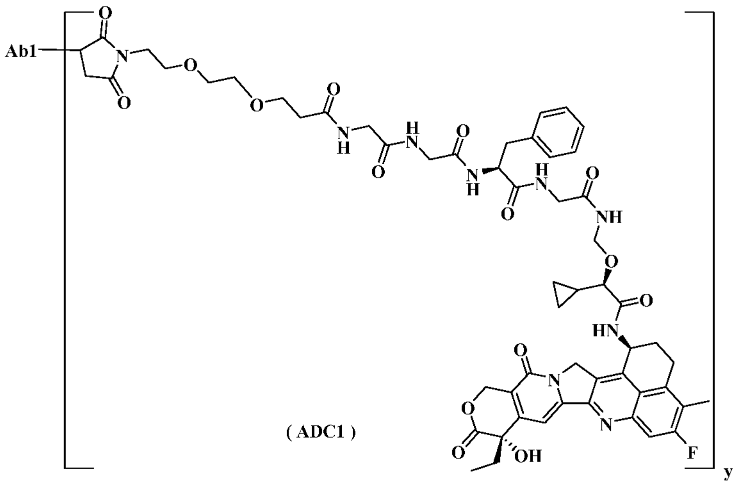

( ADC1 )

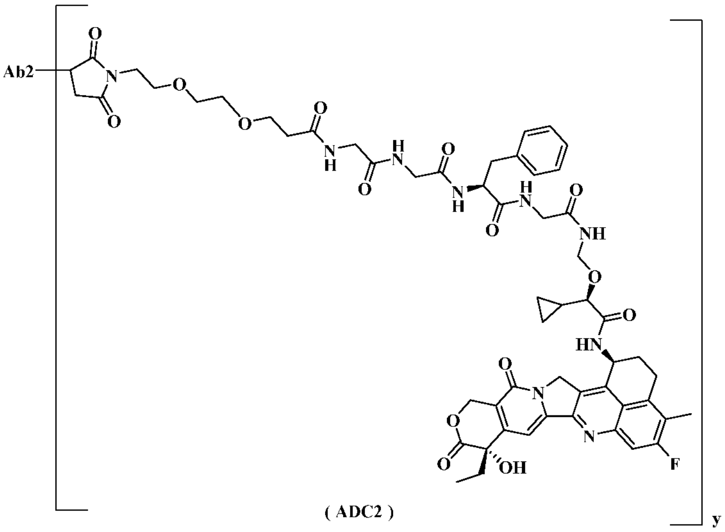

( ADC2 )

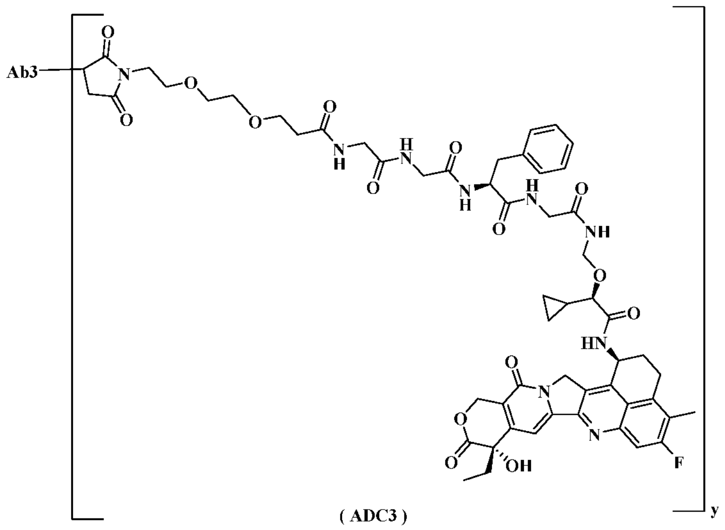

( ADC3 )

wherein y is as defined in claim 1.

**12.** A method for preparing the antibody drug conjugate of general formula (I) or the pharmaceutically acceptable salt or solvate thereof, which comprises the following steps:

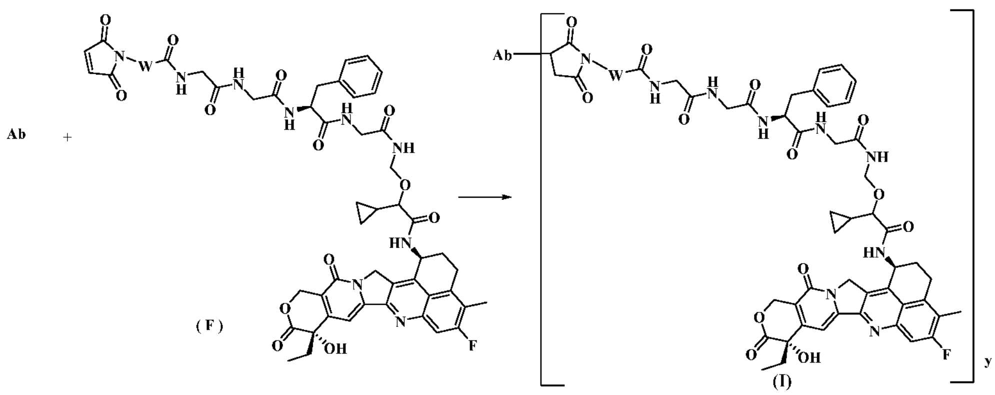

( F ) (I)

after Ab is reduced, it is subjected to coupling reaction with general formula (F) to obtain the compound of general formula (I); wherein: W, Ab, y are as defined in claim 1.

**13.** A pharmaceutical composition comprising the antibody drug conjugate according to any one of claims 1 to 11, or the pharmaceutically acceptable salt or solvate thereof, and one or more pharmaceutically acceptable excipients, diluents or carriers.

**14.** Use of the antibody drug conjugate or the pharmaceutically acceptable salt or solvate thereof according to any one of claims 1 to 11, or the pharmaceutical composition according to claim 13 in the preparation of a medicament for treating or preventing a BCMA-mediated disease or condition.

**15.** The use according to claim 14, **characterized in that** the BCMA-mediated disease or condition is cancer or autoimmune disease; wherein the cancer is preferably a cancer expressing BCMA, more preferably lymphoma, leukemia or myeloma, and the autoimmune disease is preferably selected from the group consisting of lupus erythematosus, IgA nephropathy and rheumatic arthritis.

## INTERNATIONAL SEARCH REPORT

International application No.
**PCT/CN2022/073943**

**A. CLASSIFICATION OF SUBJECT MATTER**

A61K 47/68(2017.01)i； C07K 16/18(2006.01)i； A61K 39/00(2006.01)i； A61P 35/00(2006.01)i； A61P 37/02(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, WOTXT, USTXT, CNKI, ISI web of science , baidu, pudmed, STN, genbank, 中文专利序列检索系统, 上海翰森生物医药科技有限公司, 花海清, BCMA, TNFRSF19, B细胞成熟抗原, 偶联物, conjugate, 依喜替康, exatecan, SEQ ID NOs: 3-20, 22-23

**C. DOCUMENTS CONSIDERED TO BE RELEVANT**

| Category* | Citation of document, with indication, where appropriate, of the relevant passages | Relevant to claim No. |
|---|---|---|
| PX | WO 2021018168 A1 (SHANGHAI HANSOH BIOMEDICAL CO., LTD. et al.) 04 February 2021 (2021-02-04)<br>page 2 paragraph 1 - page 8 paragraph 2 from the bottom | 1-15 |
| A | WO 2020244657 A1 (SHANGHAI HANSOH BIOMEDICAL CO., LTD. et al.) 10 December 2020 (2020-12-10)<br>claims 1-29, 34 | 1-15 |
| A | WO 2020063673 A1 (JIANGSU HENGRUI MEDICINE CO., LTD. et al.) 02 April 2020 (2020-04-02)<br>abstract, and claims 1-28 | 1-15208715994 |
| A | CN 109293772 A (BIORAY LABORATORIES INC.) 01 February 2019 (2019-02-01)<br>claims 1-10 | 1-15 |
| A | CN 109265550 A (EAST CHINA NORMAL UNIVERSITY et al.) 25 January 2019 (2019-01-25)<br>claims 1-10 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 April 2022** | **28 April 2022** |

Name and mailing address of the ISA/CN
**China National Intellectual Property Administration (ISA/CN)**
**No. 6, Xitucheng Road, Jimenqiao, Haidian District, Beijing 100088, China**
Facsimile No. **(86-10)62019451**

Authorized officer

Telephone No.

Form PCT/ISA/210 (second sheet) (January 2015)

**INTERNATIONAL SEARCH REPORT**

International application No.
**PCT/CN2022/073943**

**C. DOCUMENTS CONSIDERED TO BE RELEVANT**

| Category* | Citation of document, with indication, where appropriate, of the relevant passages | Relevant to claim No. |
|---|---|---|
| A | CN 112028996 A (NANJING BIOHENG BIOTECH CO., LTD. et al.) 04 December 2020 (2020-12-04)<br>claims 1-26 | 1-15 |

Form PCT/ISA/210 (second sheet) (January 2015)

**INTERNATIONAL SEARCH REPORT**

International application No.
**PCT/CN2022/073943**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/CN2022/073943**

| Patent document cited in search report | Publication date (day/month/year) | Patent family member(s) | Publication date (day/month/year) |
|---|---|---|---|
| WO 2021018168 A1 | 04 February 2021 | TW 202115118 A | 16 April 2021 |
| | | AU 2020322588 A1 | 03 February 2022 |
| | | CN 112585168 A | 30 March 2021 |
| | | CA 3146394 A1 | 04 February 2021 |
| WO 2020244657 A1 | 10 December 2020 | AU 2020288275 A1 | 03 February 2022 |
| | | CN 112351797 A | 09 February 2021 |
| | | KR 20220017946 A | 14 February 2022 |
| | | TW 202112398 A | 01 April 2021 |
| | | CA 3142641 A1 | 10 December 2020 |
| WO 2020063673 A1 | 02 April 2020 | JP 2022512568 A | 07 February 2022 |
| | | EP 3854816 A1 | 28 July 2021 |
| | | CN 112543771 A | 23 March 2021 |
| | | TW 202028242 A | 01 August 2020 |
| | | CA 3114474 A1 | 02 April 2020 |
| | | AU 2019351427 A1 | 15 April 2021 |
| | | US 2021347894 A1 | 11 November 2021 |
| | | BR 112021004829 A2 | 08 June 2021 |
| | | KR 20210068457 A | 09 June 2021 |
| CN 109293772 A | 01 February 2019 | None | |
| CN 109265550 A | 25 January 2019 | None | |
| CN 112028996 A | 04 December 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202110123809 A


### Non-patent literature cited in the description


- *J. Biol. Chem,* 1968, vol. 243, 3558
- **HOLLIGER ; HUDSON.** *Nat. Biotechnol.,* 2005, vol. 23, 1126-1136
- **NOVAGEN.** *TB326 Rev.B,* vol. 0503